# EUROPEAN PATENT SPECIFICATION

(11) **EP 4 429 686 B1**
(45) Date of publication and mention of the grant of the patent: **02.07.2025**
(21) Application number: 22836184.6
(22) Date of filing: 16.12.2022
(51) Int. Cl.: A61K 35/745, A61K 35/747, A61P 25/22, A61P 25/24

(54) **POSTBIOTIC COMPOSITIONS COMPRISING B. LONGUM CECT-7347 AND L. RHAMNOSUS CECT-8361 AND THERAPEUTIC USES THEREOF**
POSTBIOTISCHE ZUSAMMENSETZUNGEN ENHALTEND B. LONGUM CECT. 7347 UND L. RHAMNOSUS CECT. 8361, UND IHRE THERAPEUTISCHE ANWENDUNGEN
COMPOSITIONS POSTBIOTIQUES COMPRENANT B. LONGUM CECT-7347 ET L. RHAMNOSUS CECT-8361 ET LEURS UTILISATIONS THÉRAPEUTIQUES

(30) Priority: 16.12.2021 EP 21383147
(43) Date of publication of application: 18.09.2024
(73) Proprietor: Biopolis, S.L., 46980 Paterna (Valencia) (ES)
(72) Inventor: CHENOLL CUADROS, Maria Empar, 46185 La Pobla de Vallbona (Valencia) (ES); MARTORELL GUEROLA, Patricia, 46220 Picassent (Valencia) (ES); TORTAJADA SERRA, Marta, 46015 Valencia (ES); RAMÓN VIDAL, Daniel, 46183 La Eliana (Valencia) (ES); ROJAS MARTÍNEZ, Antonia, 46117 Bétera (Valencia) (ES); BALAGUER VIDAL, Ferran, 46006 Valencia (ES); LLOPIS PLA, Silvia, 46839 Guadassèquies (València) (ES); MARTÍNEZ ÁVILA, Roberto, 46360 Buñol (Valencia) (ES); ROBLES RODRÍGUEZ, Vanesa, 24007 León (ES); FERNÁNDEZ RIESCO, Marta, 24008 León (ES)
(74) Representative: Pons
(86) International application number: PCT/EP2022/086359
(87) International publication number: WO 2023/111270

(56) References cited:
- EP-A1- 3 222 282
- EP-A1- 3 272 396
- EP-A1- 3 851 115
- WO-A1-2018/002238
- WO-A1-2018/220429
- WO-A1-2019/038449
- MARTORELL PATRICIA ET AL: "Heat-Treated Bifidobacterium longum CECT-7347: A Whole-Cell Postbiotic with Antioxidant, Anti-Inflammatory, and Gut-Barrier Protection Properties", ANTIOXIDANTS, vol. 10, no. 4, 30 January 2021 (2021-01-30), pages 536, XP055910626, ISSN: 2076-3921, DOI: 10.3390/antiox10040536
- SALMINEN SEPPO ET AL: "The International Scientific Association of Probiotics and Prebiotics (ISAPP) consensus statement on the definition and scope of postbiotics", NATURE REVIEWS GASTROENTEROLOGY, NATURE PUBLISHING GROUP UK, LONDON, vol. 18, no. 9, 4 May 2021 (2021-05-04), pages 649 - 667, XP037596739, ISSN: 1759-5045, [retrieved on 20210504], DOI: 10.1038/S41575-021-00440-6
- JOSÉ MOISÉS LAPARRA ET AL: "Bifidobacterium longum CECT 7347 Modulates Immune Responses in a Gliadin-Induced Enteropathy Animal Model", PLOS ONE, vol. 7, no. 2, 10 February 2012 (2012-02-10), pages e30744, XP055326243, DOI: 10.1371/journal.pone.0030744

## Description

The present invention is defined in claims 1 to 12, relating to a postbiotic composition and its use in the treatment and/or prevention of anxiety disorders

### BACKGROUND ART

There is growing evidence that the bidirectional communication between the brain and the gut, called "the gut-brain axis", plays a potential role in the maintenance of the brain function as well as the gut homeostasis. Research suggests that distinct types of microbes, such as orally administered probiotics, may modify the gut-brain axis and control the stress response, suggesting that probiotics may have a therapeutic role in the management of mood disorders, such as depression or anxiety disorders, by regulating "the microbiota-gut-brain axis".

A number of viable probiotic strains and mixtures have been reported to have effects on anxiety and other behavioural indicators. However, there is very limited evidence on positive outcomes on anxiety related to the use of non-viable, inanimate, inactivated or heat-treated forms of microorganisms, including but not limited to, inanimate forms of probiotics, all of them known as postbiotics, also described as ghost probiotics or paraprobiotics.

The use of these non-viable microbial strains is extremely relevant to their industrial application, enabling the use of the product in challenging environments and the use of harsh matrixes or processes that would damage cell viability.

WO2019038449 A1 describes a pharmaceutical composition comprising non-viable *Bifidobacterium longum,* optionally in combination with *Lactobacillus mucosae* for use in reducing stress, anxiety and depression and for use in treating mood disorders, insomnia, delusional disorder, obsessive compulsive disorder, migraine, cognitive disorder, and attention disorder in a subject.

WO2018220429A1 describes probiotic *Lactobacillus rhamnosus* for use in treating or preventing postnatal depression and post-natal anxiety.

WO02018002238 A1 describes a composition comprising *Lactococcus lactis* and a combination of probiotic *Bifidobacterium longum* and *Lactobacillus rhamnosus,* for use in preventing and/or treating anxiety disorders and related conditions such as phobias, social anxiety disorder, panic disorder, obsessive-compulsive disorder and posttraumatic stress disorder. The composition is a nutritional composition, an infant formula or a pharmaceutical composition

EP3222282 A1 describes probiotic compositions comprising *B.longum* CECT 7347 and/or *L. rhamnosus* CECT 8361 for use in the treatment and/or prevention of psoriasis.

EP3851115 A1 describes a probiotic composition comprising *Lactobacillus rhamnosus* CECT 8361 and *Bifidobacterium longum* CECT 7347 for use in the treatment and/or prevention of oxidative stress in a subject.

EP3272396 A1 describes a probiotic composition comprising *Bifidobacterium longum* CECT 7347 for use in the treatment and/or prevention of atopic dermatitis.

Martorell Patricia et al., "Heat-Treated Bifidobacterium longum CECT-7347: A Whole-Cell Postbiotic with Antioxidant, Anti-Inflammatory, and Gut-Barrier Protection Properties", ANTIOXIDANTS, vol. 10, no. 4 (2021) describes heat-treated *Bifidobacterium longum* CECT 7347 (postbiotic) as having antioxidant, anti-inflammatory, and gut-barrier protection properties.

Salminen Seppo et al., "The International Scientific Association of Probiotics and Prebiotics (ISAPP) consensus statement on the definition and scope of postbiotics", NATURE REVIEWS GASTROENTEROLOGY, NATURE PUBLISHING GROUP UK, LONDON, vol. 18, no. 9, (2021), pages 649-667, discloses a postbiotic definition as a preparation of inanimate microorganisms and/or their components that confers a health benefit on the host.

José Moisés Laparra et al., "Bifidobacterium longum CECT 7347 Modulates Immune Responses in a Gliadin-Induced Enteropathy Animal Model", PLOS ONE, vol. 7, no. 2, (2012) describes that *Bifidobacterium longum* CECT 7347 modulates immune responses in a gliadin-induced enteropathy animal model.

Therefore, there is a need to develop postbiotic compositions for the treatment of anxiety disorders.

### DESCRIPTION OF THE INVENTION

The authors of the present invention have discovered that prolonged ingestion of a mixture of non-viable *Lactobacillus rhamnosus* and *Bifidobacterium longum* strains results in a positive outcome in a *Danio rerio* (zebrafish) based model of behaviour and anxiety. Surprisingly, the best results are obtained with the heat-treated postbiotic blend, rather than with each of the species alone, either viable or non-viable, or the viable probiotic blend itself.

Furthermore, comparative assays with different combinations of heat-treated *Lactobacillus rhamnosus* (*L. rhamnosus*) and *Bifidobacterium longum* (*B. longum*) strains were performed in a *Caenorhabditis elegans* (*C. elegans*) anxiety model, showing a significantly improved reduction of the anxious behaviour associated to the treatment with heat-treated *B. longum* strain CECT 7347 (also named as HT-ES1 or HT-IATA-ES1) and *L. rhamnosus* strain CECT 8361 (also named as HT-BPL15 or HT-BPL0015) blend compared to the combination of other heat-treated strains belonging to the *B. longum* and *L. rhamnosus* species (Example 3).

Thus, in an aspect, the invention relates to a postbiotic composition ("postbiotic composition of the invention" or "composition of the invention") comprising non-viable *Bifidobacterium longum* strain CECT 7347 and non-viable *Lactobacillus rhamnosus* strain CECT 8361.

*L. rhamnosus* CECT 8361 (BPL15 or BPL0015) was isolated from faeces of a healthy and breastfeeding child less than three (3) months old. This strain was deposited on 27 May 2013 under the Budapest Treaty in the Spanish Type Culture Collection as an International Depositary Authority (based in Building 3 CUE, Parc Cientific Universitat de Valencia, C/ Catedrático Agustin Escardino, 9, 46980 Paterna (Valencia) SPAIN). The deposit number assigned was CECT 8361.

*B. longum* CECT 7347 (ES1 or IATA-ES1) was isolated from faeces of a healthy breastfeeding child less than three (3) months old and deposited on 20 December 2007 under the Budapest Treaty in the Spanish Type Culture Collection as the International Depository Authority (based in Building 3 CUE, Parc Cientific Universitat de Valencia, C/ Catedrático Agustin Escardino, 9, 46980 Paterna (Valencia) SPAIN). The deposit number assigned was CECT 7347.

As used herein, the term "postbiotic composition" relates to any compositions comprising at least one postbiotic, including inanimate bacteria or its components. According to the definition provided by International Scientific Association of Probiotics and Prebiotics (ISAPP), a postbiotic is a "preparation of inanimate microorganisms and/or their components that confers a health benefit on the host" (Sallines et al. 2021 Nature Rev Gastroenterol Hepatol. 18(9):649-667). Conversely, and according to the World Health Organization, probiotics are "live microorganisms that, when administered in adequate amounts, confer a health benefit on the host".

The past years have seen a research focus on members of the gut microbiota that exhibit health-promoting effects such as protection of the host against pathogens by competitive exclusion or modulation of the immune system. The term "gut microbiota" relates to the diverse microbial community evolved to adapt and survive in the human gastrointestinal tract. The gut microbiota interacts with their human host in a variety of ways; the microbes may be being innocuous commensals, opportunistic pathogens or health-promoting or probiotic microorganisms. Research into the activities of the latter has increased substantially over the last 20 years, with probiotic agents being investigated in many clinical and animal model-based studies.

In the present invention, the postbiotic composition comprises non-viable bacteria belonging to *B. longum* and *L. rhamnosus.* The taxonomy of both species is detailed below.

| **Kingdom** | **Class** | **Order** | **Family** | **Genus** | **Species** |
|---|---|---|---|---|---|
| *Bacteria* | *Firmicutes* | *Actinobacteria* | *Bifidobacteriaceae* | *Bifidobacterium* | *B. longum* |
| *Bacteria* | *Firmicutes* | *Lactobacillales* | *Lactobacillaceae* | *Lactobacillus* / *Lacticaseibacillus* | *L. rhamnosus* |

Members of the genus *Bifidobacterium* are among the first microbes to colonize the human gastrointestinal tract (GIT) and are believed to exert positive health benefits on their host. Bifidobacteria have been commercially exploited as probiotic agents due to their associated health benefits and to being generally recognised as safe. Due to their purported health-promoting properties, these bacteria have been incorporated into many functional foods as active ingredients.

Bifidobacteria naturally occur in a range of ecological niches that are either directly or indirectly connected to the animal GIT, such as the human oral cavity, the insect gut and sewage. To be able to survive in these particular ecological niches, Bifidobacterial must possess specific adaptations to be competitive.

*Bifidobacterium* are gram-positive, non-motile, and often branched anaerobic bacteria. They are one of the major genera of bacteria that make up the GIT microbiota in mammals, and comprise over 30 different species. Among them, *B. longum* is a catalase-negative, branched rod-shaped bacterium.

*Lactobacillus* (currently known as *Lacticaseibacillus*) is the largest genus of the family *Lactobacillaceae.* Before its reclassification into 23 different genera (Zheng et al. 2020 Int J Syst Evol Microbiol. 70(4):2782-2858), it consisted of 196 validly published species, commonly isolated not only from environments associated with fermented food, such as fruits, meat, sourdough, vegetables, and wine, but also from the gastrointestinal and vaginal tracts of humans and animals. Currently, they are widely applied in fields related to food, feed, pharmaceuticals and biotechnology; for example, they are used as dairy starters, probiotics, vaccine carriers and silage inoculants.

Lactobacilli are gram-positive, rod-shaped, facultatively anaerobic or microaerophilic, non-spore-forming, acid-tolerant, and catalase-negative bacteria with DNA G+C content between 46 and 58 mol%. *Lactobacillus casei, Lactobacillus paracasei,* and *Lactobacillus rhamnosus* are phylogenetically and phenotypically closely related, and were formerly regarded as the *L. casei* group (Zheng et al. 2020 Int J Syst Evol Microbiol. 70(4):2782-2858). The probiotic strains that are part of this group are used worldwide in fermented dairy products or food supplements and as probiotics to enhance host health.

The postbiotic composition of the invention comprises non-viable forms of bacteria *B. longum* and *L. rhamnosus.* As used herein, the term "non-viable", "inanimate", "inactive" or "inactivated" relates to any microorganism metabolically or physiologically inactive i.e., not retaining its metabolic activity and the ability to elongate after the administration of nutrients. Viability is independent from the capacity of the microorganism to form colonies on solid media i.e., its culturability.

Thermal processing is likely to be used in many instances to inactivate microorganisms, as there is a long history of thermal processing in the food industry. A common procedure to inactivate microorganisms is heat treatment. Heat is lethal to microorganisms, but each species has its own particular heat tolerance. During a thermal destruction process, such as pasteurization, tyndallisation and autoclaving, the rate of destruction is logarithmic, as is their rate of growth. Thus, bacteria subjected to heat are killed at a rate that is proportional to the number of organisms present. The process is dependent both on the temperature of exposure and the time required at this temperature to accomplish to desired rate of destruction.

In some embodiments of the invention, the non-viable *Bifidobacterium longum* and *Lactobacillus rhamnosus* strains are heat-treated. Thus, a preferred embodiment provides the composition of the invention, wherein the non-viable *B. longum* strain CECT 7347 is heat-treated and the non-viable *Lactobacillus rhamnosus* strain CECT 8361 is heat-treated.

The present invention also contemplates those bacterial strains derived from *B. longum* CECT 7347 and *L. rhamnosus* CECT 8361 strains ("parental strains") that may be part of the postbiotic composition of the invention as they retain the ability to reduce and/or improve the symptoms of anxiety disorders in the subject.

Examples of bacterial strains derived from the parental strains comprised within the composition of the invention include genetically modified organisms which show variations in their genome compared to the genome of the strains of the invention, wherein the mutations do not affect the ability of strains and/or their components to reduce and/or improve the symptoms of anxiety disorders in the subject.

Strains derived from *B. longum* CECT 7347 and *L. rhamnosus* CECT 8361 may be naturally or intentionally produced by mutagenesis methods known in the art, such as, but not limited to, growing the parent strain in the presence of mutagenic agents or stressors or genetic engineering directed to the modification, deletion and/or insertion of specific genes.

In some embodiments, the composition of the invention contemplates genetically modified organisms derived from *B. longum* CECT 7347 and *L. rhamnosus* CECT 8361 strains that retain the ability to prevent, reduce and/or improve the symptoms of anxiety disorders in a subject and, therefore, said composition useful in the treatment and/or prevention of said disorders.

In some specific embodiments, the present invention also contemplates bacterial components, metabolites and molecules secreted by *B. longum* CECT 7347 and/or *L*. *rhamnosus* CECT 8361 strains and/or compositions comprising said components, as well as their use for the treatment and/or prevention of anxiety disorders.

Bacterial components include, without limitation, components of the cell wall (by way of non-limiting example, peptidoglycan); nucleic acids; membrane components; and proteins, lipids, carbohydrates, and combinations thereof (such as lipoproteins, glycolipids or glycoproteins). Metabolites may include any molecule produced or modified by the bacterium as a result of its metabolic activity during growth, its use in technological processes or during its storage. Examples of these metabolites include, but are not limited to, organic and inorganic acids, proteins, peptides, amino acids, enzymes, lipids, carbohydrates, lipoproteins, glycolipids, glycoproteins, vitamins, salts, minerals or nucleic acids. Secreted molecules include any molecule secreted or released to the outside by the bacterium during growth, its use in technological processes (for example, food processing or drugs) or during its storage. Examples of these molecules include, but are not limited to, organic and inorganic acids, proteins, peptides, amino acids, enzymes, lipids, carbohydrates, lipoproteins, glycolipids, glycoproteins, vitamins, salts, minerals or nucleic acids.

The composition of the invention may be formulated for pharmaceutical administration, i.e., forming part of pharmaceutical products to be administered to the subject by any means of administration; and/or for food administration, i.e., forming part of the foods or nutritional supplements consumed in the subject's diet. Thus, in some embodiments, the composition of the invention is a pharmaceutical composition ("pharmaceutical composition of the invention") and/or a nutritional composition ("nutritional composition of the invention").

The composition of the invention comprises non-viable *B*. *longum* CECT 7347 and *L*. *rhamnosus* CECT 8361 (and/or strains and/or cellular components derived therefrom) at any suitable concentration.

Additionally, the composition of the invention may comprise (i) one or more components or compounds having any biological, pharmacological and/or veterinary useful activity, as an activity useful in the prevention and/or treatment of anxiety disorders in a subject; and/or (ii) one or more components that, upon administration to a subject, may increase, enhance and/or promote the activity of the postbiotics included in the composition of the invention. As understood by those skilled in the art, the additional components or compounds must be compatible with the effect exerted by the non-viable strains of the composition of the invention.

The term "pharmaceutical composition" also encompasses veterinary compositions.

In some embodiments, the pharmaceutical composition further comprises a pharmaceutically acceptable carrier and/or excipient.

The term "excipient" refers to a substance that helps the absorption of any components or compounds of the composition of the invention, namely, of strains of the invention, or stabilizes the components or compounds and/or assists the preparation of the pharmaceutical composition in the sense of giving it consistency or flavours to make it more pleasant. Thus, the excipients may have the function, by way of example but not limited thereto, of binding the components (for example, starches, sugars or cellulose), sweetening, colouring, protecting the active ingredient (for example, to insulate it from air and/or moisture), filling a pill, capsule or any other presentation or a disintegrating function to facilitate dissolution of the components, without excluding other excipients not listed in this paragraph. Therefore, the term "excipient" is defined as that material that included in the galenic forms, is added to the active ingredients or their associations to enable their preparation and stability, modify their organoleptic properties or determine the physical and chemical properties of the pharmaceutical composition and its bioavailability. The "pharmaceutically acceptable" excipient must allow the activity of components or compounds of the pharmaceutical composition, that is, be compatible with the effect exerted by the strains of the invention.

The "galenic form" or "pharmaceutic form" is the configuration to which the active ingredients and excipients are adapted to provide a pharmaceutical composition or a drug. It is defined by the combination of the form in which the pharmaceutical composition is presented by the manufacturer and the form in which it is administered.

The "vehicle" or "carrier" is preferably an inert substance. Carrier functions are to facilitate the incorporation of other components or compounds, allow better dosage and administration and/or give consistency and form to the pharmaceutical composition. Therefore, the carrier is a substance used in the drug to dilute any of the components or compounds of the pharmaceutical composition of the present invention to a given volume or weight; or that even without diluting these components or compounds, it is able to allow better dosage and administration and/or give consistency and form to the drug. When the presentation is liquid, the pharmaceutically acceptable carrier is the diluent. The carrier can be natural or unnatural. Examples of pharmaceutically acceptable carriers include, without being limited thereto, water, salt solutions, alcohol, vegetable oils, polyethylene glycols, gelatine, lactose, starch, amylose, magnesium stearate, talc, surfactants, silicic acid, viscous paraffin, perfume oil, monoglycerides and diglycerides of fatty acids, fatty acid esters petroetrals, hydroxymethylcellulose, polyvinylpyrrolidone and the like.

Furthermore, the excipient and the carrier must be pharmacologically acceptable, i.e., the excipient and the carrier are permitted and evaluated so as not to cause damage to the subject to whom it is administered.

In each case the presentation of the composition will be adapted to the type of administration used. Thus, the composition may be presented in the form of solutions or any other form of clinically permissible administration and in a therapeutically effective amount. The composition of the invention can be formulated into solid, semisolid or liquid preparations, such as tablets, capsules, powders, granules, solutions, suppositories, gels or microspheres. In a particular embodiment, the composition of the invention is formulated in liquid form or in solid form.

In another particular embodiment, the solid formulation is selected from the group consisting of tablets, lozenges, sweets, chewable tablets, chewing gums, capsules, sachets, powders, gels, granules, coated particles or coated tablets, tablet, pills, troches, gastro-resistant tablets and capsules and dispersible strips and films.

In another particular embodiment, the liquid formulation is selected from the group consisting of oral solutions, suspensions, droplets, emulsions and syrups.

Likewise, various systems are known that can be used for sustained-release administration of the composition of the invention, including, for example, the encapsulation in liposomes, microbubbles, microparticles or microcapsules and the like. The suitable sustained-release forms as well as materials and methods for their preparation are well known in the state of the art. Thus, the orally administrable form of the composition of the invention is in a sustained-release form further comprising at least one coating or matrix. The sustained release coating or matrix includes, without limitation, natural semisynthetic or synthetic polymers, water-insoluble or modified, waxes, fats, fatty alcohols, fatty acids, natural, semisynthetic or synthetic plasticizers or a combination of two or more of the same. Enteric coatings can be applied using conventional processes known to those skilled in the art.

In addition to what has been described above, the present invention also encompasses the possibility that the composition of the invention may be administered to a subject together with other components or compounds, although these are not part of the composition of the invention. Examples of such components or compounds are detailed in preceding paragraphs.

In some embodiments of the present invention, the composition of the invention is a nutritional composition. The nutritional composition of the invention also comprises non-viable bacteria *B. longum* CECT 7347 and *L. rhamnosus* CECT 8361 (and/or strains and/or cellular components derived therefrom) at any suitable concentration.

In some embodiments, the composition of the invention comprises *L. rhamnosus* CECT 8361 and *B. longum* CECT 7347 (and/or strains and/or cellular components derived therefrom) at any suitable concentration.

In the event that the composition of the invention is formulated as a nutritional composition, said nutritional composition may be a food or be incorporated into a food or food product intended for human and/or animal consumption.

Thus, in some embodiments, the nutritional composition of the invention is a food or a nutritional supplement.

In some specific embodiments of the invention, the nutritional composition of the invention is a food for specific nutritional and/or functional, biological and/or physiological purposes. In some specific embodiments of the invention, the nutritional composition of the invention is a medicinal food.

In some embodiments, the nutritional composition of the invention is a nutritional or dietary supplement.

In the present invention, the term "nutritional composition" refers to any food which, regardless of providing nutrients to the subject who consumes it, beneficially affects one or more functions of the body, so as to leverage and/or provide better health and wellness. In the present invention, said nutritional composition is intended to ease, reduce, treat and/or prevent anxiety disorders.

The term "supplement", synonymous with any of the terms "dietary supplement", "nutritional supplement", "food supplement", or "alimentary supplement" or "alimentary complement" refers to products or preparations whose purpose is to supplement the normal diet of a subject consisting of sources of concentrated nutrients or other substances with a nutritional or physiological effect. In the present invention, the *B. longum* CECT 7347, *L. rhamnosus* CECT 8361 and/or any strains and/or cellular components derived from them are the "substances" with a nutritional and/or physiological effect on the subject.

The food supplement may be in single or combined form and be marketed in dosage form, i.e., in capsules, pills, tablets and other similar forms, sachets of powder, ampoules of liquids and drop dispensing bottles and other similar forms of liquids and powders designed to be taken in a single amount.

There is a wide range of nutrients and other elements that may be present in alimentary complements including, among others, vitamins, minerals, amino acids, essential fatty acids, fibre, enzymes, plants and plant extracts. Since their role is to complement the supply of nutrients in a diet, they should not be used as a substitute for a balanced diet and intake should not exceed the daily dose expressly recommended by the doctor or the nutritionist. The composition of the invention can also be part of the so-called "food for special groups", i.e., foods that meet specific nutritional needs.

Examples of foods that may comprise the composition of the invention include, but are not limited to, feed, dairy products, vegetable products, meat products, snacks, chocolates, beverages, baby food, cereals, fried foods, industrial bakery products and biscuits. Examples of dairy products include, but are not limited to, products derived from fermented milk (for example, but not limited to, yogurt or cheese) or non-fermented milk (for example, but not limited to, ice cream, butter, margarine or whey). The vegetable product is, for example, but not limited to, a cereal in any form of presentation, fermented (for example, soy yogurt, oat yogurt, etc.) or unfermented, and a snack. The beverages include, but are not limited to, non-fermented milk.

In some embodiments, the food product or food is selected from the group consisting of fruit or vegetable juices, ice cream, infant formula, milk, yogurt, cheese, fermented milk, powdered milk, cereals, baked goods, milk-based products (such as milkshakes or smoothies), meat products and beverages.

In some embodiments, the composition of the invention may comprise other microorganisms in addition to *B*. *longum* CECT 7347 and *L. rhamnosus* CECT 8361.

As understood by the person skilled in the art, B. *longum* and *L. rhamnosus* strains have to be present, jointly or separately, in the composition of the invention in a therapeutically effective amount in order to exert their effect, such as the effect of easing, reducing, treating and/or preventing anxiety disorders.

Within the context of the present invention, a "therapeutically effective amount" is any amount of the component or compound of the nutritional or pharmaceutical composition, which, when administered to a subject, is sufficient to produce the desired effect. Said component or compound of the nutritional or pharmaceutical composition refers to the bacterial strains *B. longum* CECT 7347 and *L. rhamnosus* CECT 8361. The therapeutically effective amount may vary depending on, for example, the age, body weight, general health, sex and diet of the subject, as well as on the mode and time of administration, the excretion rate or any potential co-treatment with other drugs. For the avoidance of doubt, said drugs may be prescribed for the prevention and treatment of anxiety disorders or for any other condition.

In some embodiments of the composition of the invention, the total concentration of microorganisms of *B. longum* CECT 7347 and *L. rhamnosus* CECT 8361 is between 10³ and 10¹² cfu or cells, preferably 10⁹ cfu or cells.

In some specific embodiments of the composition of the invention, the amount of microorganisms of *B. longum* CECT 7347 and *L. rhamnosus* CECT 8361 is between 10³ and 10¹² cfu or cells, preferably 10⁹ cfu or cells. In some specific embodiments, the nutritional composition of the invention is a food or a nutritional supplement for humans.

In some specific embodiments, the nutritional composition of the invention is a food or a nutritional supplement for farm animals and/or pets.

In some specific embodiments, the nutritional composition of the invention is a food or a nutritional supplement for prevention of anxiety disorders in healthy humans.

In some specific embodiments, the nutritional composition of the invention is a food or a nutritional supplement for prevention of anxiety disorders in healthy farm animals and/or pets.

In another preferred embodiment, the composition of the invention further comprises a microorganism or their components selected from the group consisting of *Bacillus* sp., *Lactobacillus* sp., *Streptococcus* sp., *Bifidobacterium* sp., *Saccharomyces* sp., *Kluyveromyces* sp. and combinations thereof.

As stated above, the use of probiotics for different conditions or to improve general health is well established; however, that is not the case with the use of postbiotics. In particular, whilst a number of probiotic strains and mixtures have been reported to have effects on anxiety and other behavioural indicators, there has not been much evidence on any positive outcomes on anxiety related to the use of postbiotics. The present application provides data demonstrating the efficacy of a postbiotic composition comprising non-viable *L. rhamnosus* and *B. longum* strains in the treatment of anxiety disorders for the first time.

Thus, in other aspect, the present invention relates to the composition of the invention, for use as a medicament.

The term "medicament", as used herein, refers to any composition/substance used for the prevention, diagnosis, alleviation, treatment or cure of disease in a subject or which may be administered to a subject for the purpose of restoring, correcting or modifying physiological functions by exerting a pharmacological, immunological or metabolic action.

In other aspect, the present invention relates to the composition of the invention, for use in the treatment and/or prevention of anxiety disorders in a subject.

As used herein, the term "to treat" or "treatment" comprises: inhibiting the disease or pathological condition, i.e., stopping its development; relieving the disease or pathological condition, i.e., causing regression of the disease or pathological condition; and/or stabilizing the disease or pathological condition in a subject. In the present invention, the disease or pathological condition is at least one anxiety disorder.

As used herein, the term "prevention" means the avoidance of occurrence of the disease or pathological condition in a subject, particularly when the subject has predisposition for the pathological condition, but has not yet been diagnosed. In the present invention, the disease or pathological condition is at least one anxiety disorder.

Within the context of the present invention, the term "subject" relates to any animal from any species. Examples of subjects include, but are not limited to: Animals of commercial interest such as birds (hens, ostriches, chicks, geese, partridges, etc.), rabbits, hares, pets (dogs, cats, etc.), sheep, goat cattle (goats, etc.), swine (boars, pigs, etc.), equine livestock (horses, ponies, etc.), and cattle (bulls, cows, oxen, etc.); animals of hunting interest, such as stags, deer, reindeer, etc.; and humans.

In some embodiments, the subject is a mammal; in some preferred embodiments, the mammal is a human being of any race, sex or age.

In some other preferred embodiments, the mammal is a farm animal, including but not limited to, cows, horses, goats, llamas, sheep, pigs, chickens, or any animal raised or bred for commercial purposes. In some other preferred embodiments, the mammal is a pet, including but not limited to, dogs, cats or rabbits.

In some embodiments, the composition of the invention is administered to a subject through the diet.

In some embodiments of the invention, the administration regime of the composition of the invention is at least once daily; twice daily; or three times a day, one with each main food intake (breakfast and/or lunch and/or dinner).

The use of postbiotics is extremely relevant to their industrial applicability, specifically for their manufacturing; it allows not only the use of the product in challenging environments subjected to heat or moisture that would impair stability of traditional probiotics, but also the use of harsh matrixes or processes (e.g., feed extrusion) that would damage probiotic cell viability.

Furthermore, the use of both postbiotics and probiotics may have a number of benefits over traditional treatments of anxiety disorders, avoiding typical side effects of standard medication, such as Fluoxetine, and including but not limited to insomnia, muscle tension, nausea, and sexual dysfunction.

As explained above, an aspect of the invention relates to postbiotic compositions comprising non-viable *B. longum* CECT 7347 and *L. rhamnosus* CECT 8361 strains for use in the treatment and/or prevention of anxiety disorders.

Anxiety is an emotion characterized by feelings of tension, worried thoughts and physical changes like increased blood pressure. Whilst anxiety is a normal reaction to stress and can be beneficial in some situations, anxiety disorders differ from normal feelings of nervousness or anxiousness, and involve excessive fear or anxiety. Anxiety may be experienced not only by humans but also by other animals, in particular by other non-human mammals.

Anxiety disorders are the most common of mental disorders in humans and affect nearly 30% of adults at some point in their lives. Anxiety disorders include, but without limitations, generalized anxiety disorder, panic disorder, specific phobia, agoraphobia, social anxiety disorder (previously called social phobia), noise aversion, and separation anxiety disorder.

Generalized anxiety disorder involves persistent and excessive worry that interferes with daily activities. It may be accompanied by physical symptoms, such as restlessness, feeling on edge or easily fatigued, difficulty concentrating, muscle tension or problems sleeping.

Panic disorder is usually characterized by recurrent panic attacks, an overwhelming combination of physical and psychological distress with rather sever symptoms. The attacks may be expected, such as a response to a feared object, or unexpected, apparently occurring for no reason. They may occur with other mental disorders such as depression or post-traumatic stress disorder (PTSD).

A specific phobia is excessive and persistent fear of a specific object, situation or activity that is generally not harmful.

Agoraphobia is the fear of being in situations where escape may be difficult or embarrassing, or help might not be available in the event of panic symptoms. The fear is out of proportion to the actual situation and lasts generally six months or more and causes problems in functioning.

Social anxiety disorder is characterized by a significant anxiety and discomfort about being embarrassed, humiliated, rejected or looked down on in social interactions. The fear or anxiety causes problems with daily functioning and lasts at least six months.

The causes of anxiety disorders are currently unknown but likely involve a combination of factors including genetic, environmental, psychological and developmental.

Although each anxiety disorder has unique characteristics, most respond well to psychotherapy and medications. Whilst medications do not cure anxiety disorders, they provide significant relief from symptoms. The most commonly used medications are anti-anxiety medications (generally prescribed only for a short period of time) and antidepressants. Beta-blockers, used for heart conditions, are sometimes used to control physical symptoms of anxiety.

Within the present invention, the term "anxiety disorders" includes not only those anxiety disorders described above, but also related conditions, including but not limited to: post-traumatic stress disorder (PTSD); acute stress disorder; obsessive-compulsive disorder; and adjustment disorders.

PTSD is a psychiatric disorder that may occur in people who have experienced or witnessed a traumatic event such as a natural disaster, a serious accident, a terrorist act, etc. or who have been threatened with death, sexual violence or serious injury. It can occur in all people, of any ethnicity, nationality or culture, and at any age. People with PTSD have intense, disturbing thoughts and feelings related to their experience that last long after the traumatic event has ended. They also experience sadness, fear or anger, and they may feel detached or estranged from other people.

Acute stress disorder occurs in reaction to a traumatic event, just as PTSD, and the symptoms are similar. However, the symptoms occur between three days and one month after the event, causing cause major distress and problems in the subject's daily lives. About half of people with acute stress disorder go on to have PTSD. Psychotherapy and medication can both help control symptoms and prevent development of the acute stress disorder into PTSD.

Obsessive-compulsive disorder (OCD) is a disorder in which people have recurring, unwanted thoughts, ideas or sensations (obsessions) that make them feel driven to do something repetitively (compulsions). These compulsions can significantly interfere with a person's daily activities and social interactions, cause significant distress, and impair work or social functioning.

Adjustment disorder also occurs in response to a stressful life event (or events). The emotional or behavioural symptoms a person experiences in response to the stressor are more severe or more intense than what would be reasonably expected for the type of event that occurred. Symptoms may include sadness, hopelessness, and physical manifestations like tremors, palpitations, and headaches. These symptoms cause significant distress or problems functioning in important areas of the subject's life, and begin usually within three months of the stressful event; they last no longer than six months after the stressor or its consequences have ended. The disorder is typically treated with psychotherapy.

As stated above, animals, in particular mammals, may also develop anxiety. For example, separation anxiety is commonly seen in pets. Separation anxiety is triggered when dogs or cats are separated from their guardians; escape attempts by the animals are often extreme and can result in self-injury. There is evidence that social stress may also play a critical role in pets' welfare. Furthermore, there is evidence that social stress, transportation and high level of density in farms, may also play a critical role in farm animal disease prevention and animal farm's welfare, and may be a mediator by which common management practices can increase disease risk. Social factors, including deprivation of social contact ('social isolation'), reducing space allowance ('crowding') and disturbing social order ('social instability') trigger physiological and behavioural indicators of stress in livestock.

In other aspect, the present invention encompasses a method for use in the treatment or prevention of anxiety disorders in a subject ("method for use in the treatment or prevention of the invention") comprising administering the composition of the invention to a subject.

Other aspect of the invention relates to the first medical use of the composition of the invention. The manufacture of a medicament for the treatment or prevention of anxiety disorders in a subject is herein described. All particular terms, definitions and embodiments of previous aspects of the invention are applicable to the method for use in treatment of and use of the invention.

In addition to related uses in the treatment and/or prevention of anxiety disorders, the composition herein described can be also used in ameliorating non-pathological anxiety.

As stated above in the present document, anxiety is a normal reaction to stress and can be beneficial in some situations. Therefore, a distinction can be made between normal feelings of nervousness or anxiousness and anxiety disorders.

Anxiety can be a typical reaction to stress and new situations for an individual, encompassing feelings of, without being limited to, worry, unease, or nervousness in a subject.

The composition herein described can be also used in the amelioration of nervousness and/or worry in a subject.

Furthermore, the composition of the invention can be used as a food supplement. Thus, other aspect of the invention relates to the use of the composition of the invention as a food supplement.

The term "food supplement" have been defined above, and its definition, as well as related particular embodiments, apply equally herein.

Throughout the description and claims the word "comprise" and its variants are not intended to exclude other technical characteristics, additives, components or steps.

### DESCRIPTION OF THE DRAWINGS

**Figure 1****.** Animal weight (mean ± SEM) in each experimental group at the beginning of the experiment, t=0.
**Figure 2****.** Image of the template generated with Noldus Ethovision^{®} used for the analysis of the swimming of the specimens with two arenas upper and lower.
**Figure 3*****.*** Animal length and weight (mean ± SEM) in each experimental group at t= 2 mpi and t=4 mpi. Asterisks show statistically significant differences *(p>0.050).
**Figure 4****.** Total distance swam and fish speed (mean ± SEM) in each experimental group at t= 2 mpi and t=4 mpi.
**Figure 5****.** Zone preference expressed as time in seconds spent in the upper zone of the tank (mean ± SEM) and normalized percentages during the NTT recording (mean ± SEM) in each experimental group at t= 2 mpi and t=4 mpi.
**Figure 6****.** % of fish (dark dots) spending less than 30 s in the upper zone during the NTT at (A) 2 months (2 mpi) and (B) 4 months (4 mpi) after the beginning of the experiment.
**Figure 7****.** Latency (s) to the first entry to the upper zone. Asterisks indicate fish spending all the NTT in the upper area of the tank. Hashes indicate fish spending all the NTT in the lower area of the tank.
**Figure 8****.** Merge heatmaps of the all the trajectories of each experimental group at 2 mpi sampling.
**Figure 9****.** Merge heatmaps of the all the trajectories of each experimental group at 4 mpi sampling.
**Figure 10****.** Representation of time to respond of *C*. *elegans* to octanol at control condition (with OP50), control Anxiety condition (with deprivation of food) and anxious nematodes treated with the mix heat-treated *L. rhamnosus* CECT8361 and heat-treated *B*. *longum* CECT7347 (HT-BPL15+HT-ES1).
**Figure 11****.** Analysis of dispersion by quantifying the nematode's distribution in agar plates. Nematodes were submitted to a stress (cholinergic stimulatory drug) and treated with the mix heat-treated *L. rhamnosus* CECT8361 and heat-treated *B*. *longum* CECT7347 (HT-BPL15+HT-ES1). Different grey scale indicates the different position (cm) of nematodes from the center of plate.
**Figure 12****.** Effect of the blend HT-ES1+HT-BPL15 (10⁸ cells) and other blends formulated with alternative strains (BPL33, BPLA3, BPL31 and BPL8) on avoidance behavior (anxiety) in *C*. *elegans.* One-way ANOVA was applied. Data are the average of two independent experiments. **p<0,01.

### Examples

### Example 1. EFFECT OF ORAL ADMINISTRATION OF SIX DIFFERENT PROBIOTIC/POSTBIOTIC SUPPLEMENTS ON ADULT ZEBRAFISH BEHAVIOUR

### Materials and Methods

Evaluation of the effect of oral administration of six different probiotic supplements* on adult *Danio rerio* (zebrafish) behaviour using the Novel Tank Test (NTT). Analysis of fish swimming pattern at individualized level and generation of a report with results and conclusions.
* Blend Formulation 004009
   HT (Heat Treated) Blend Formulation 004009
   *Bifidobacterium longum* ES1 (CECT 7347)
   HT *Bifidobacterium longum* ES1
   *Lactobacillus rhamnosus* BPL15 (CECT 8361)
   HT *Lactobacillus rhamnosus* BPL15

All procedures described in the present report were approved by the institutional Animal Care and Use Committee of the University of León (Spain). All animals were standard manipulated in accordance with European Union Council Guidelines (2010/63/EU), following Spanish regulations (RD/2013) for the use of laboratory animals.

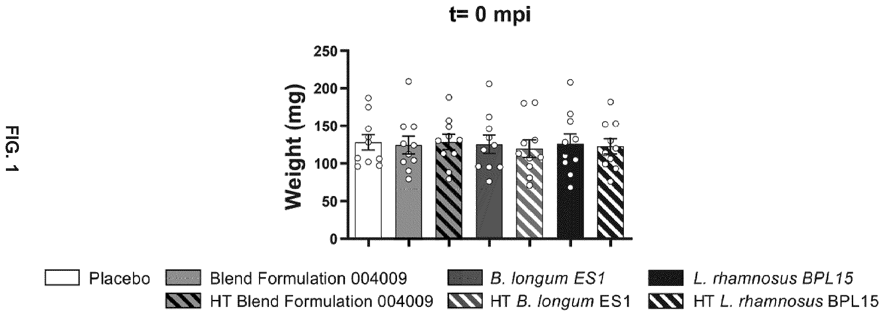

A batch of 70 4-month post fertilization (mpf) zebrafish (AB strain) siblings were homogeneously divided into 7 groups (n=10) considering fish weight (Figure 1, Table 1).

**Table 1.- Weight data (mg) at the beginning of the experiment.**

| **P** | **Blend** | **HTBlend** | **ES1** | **HTES1** | **BPL15** | **HTBPL15** | |
|---|---|---|---|---|---|---|---|
| 175 | 149 | 113 | 162 | 180 | 182 | 208 | |
| 128 | 209 | 188 | 134 | 127 | 106 | 166 | |
| 146 | 104 | 131 | 146 | 131 | 120 | 110 | |
| 187 | 126 | 157 | 206 | 181 | 152 | 128 | |
| 107 | 112 | 130 | 118 | 113 | 114 | 156 | |
| 102 | 90 | 149 | 124 | 81 | 76 | 101 | |
| 106 | 79 | 136 | 76 | 71 | 99 | 68 | |
| 97 | 124 | 88 | 95 | 96 | 153 | 85 | |
| 135 | 149 | 79 | 96 | 108 | 93 | 132 | |
| 96 | 102 | 113 | 95 | 107 | 130 | 105 | |
| | | | | | | | |

| | **P** | **Blend** | **HTBlend** | **ES1** | **HTES1** | **BPL15** | **HTBPL15** |
|---|---|---|---|---|---|---|---|
| Mean | 127,9 | 124,4 | 128,4 | 125,2 | 119,5 | 122,5 | 125,9 |
| Std. Deviation | 32,74 | 37,54 | 32,33 | 38,79 | 37,04 | 32,2 | 41,67 |
| Std. Error of Mean | 10,35 | 11,87 | 10,22 | 12,27 | 11,71 | 10,18 | 13,18 |
| | | | | | | | |

Each fish was anesthetized in 110 mg/L buffered tricaine methane sulfonate (MS222) and individually tagged with visible implant elastomers (Northwest Marine Technology, WA, USA) to individual identification and animal tracking. Zebrafish were maintained in 3 L tanks with constant water exchanged from a recirculation system equipped with mechanical, chemical and biological filters. The water was kept at a mean temperature of 26 °C, and the room photoperiod consisted on a 14/10 light/dark cycle.

The probiotic and/or postbiotic supplements and the placebo were provided in capsules containing 10⁹ cfu or cells. The content of the capsule was split into two and in order to guarantee the ingestion, each dose (half of the content) was provided to experimental males in rearing water 30 min before each routine feeding (twice a day). These feeding conditions were maintained during all the experiment (4 months). The technical personnel in charge of the maintenance, feeding and animal welfare were unaware of the contents of the capsules. The experimental groups were blindly numbered from 1 to 7.

To test the exploratory behaviour of the animals, we used a novel tank test (NTT). This test enables the study of the strength of the geotaxis escape diving instinct of a fish under specific experimental conditions since it has a conflict between "safe" diving behaviour and "exploration" swimming behaviour. Under normal controlled laboratory conditions, fish generally tend to spend more time at the bottom of the tank in the first minutes of monitoring, showing a higher rate of erratic movements and immobility events. After the habituation period, the animals gradually explore the upper areas of the water column.

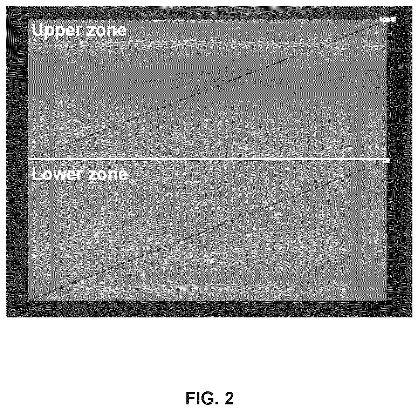

The protocol was performed twice: 2 and 4 months post the start of ingestion (mpi) as following: each animal was individually placed at the bottom of a quadrangular glass aquarium (20 × 8 × 18 cm; length × width × depth; swimming volume: 3.5 L). The fish were filmed (1920 × 1080 px) during 6 min. The first minute was considered as acclimation time to the new environment and the behaviour analysis was considered for the rest 5 min. Individual swimming activity was analyzed using Noldus Ethovision^{®} tracking software, generating a virtual grid dividing the tank in 2 arenas (upper and lower; Figure 2).

For each animal we quantified the next estimates of exploratory behavior:
- Distance moved.
- Velocity.
- Preference of zone.
- Latency to the first entry in the upper zone.

Results are expressed as the mean ± standard error. Percentage data were normalized using arcsine-square root transformation. Statistical differences between mean values of each variable at 2 and 4 mpi were determined using a one-tail unpaired t-Student test (normally distributed variables) or a one tail Mann-Whitney test (non-parametric variables). All statistical analyses were performed using Prism 9 (GraphPad Software, San Diego, CA, USA). P-values < 0.0500 were considered statistically significant. Postbiotic The experiments were conducted using a Danio rerio (zebrafish) model of anxiety. The model is based on zebrafish preference for swimming close to the bottom when first placed in a novel tank. Differences in time the fish take to before they leave the bottom of the tank and start to explore other areas are considered to be a reflection of the anxiety they experience. Parameters related to the swimming patterns, mean swimming speed and distribution across the tank were analysed.

### Biometry

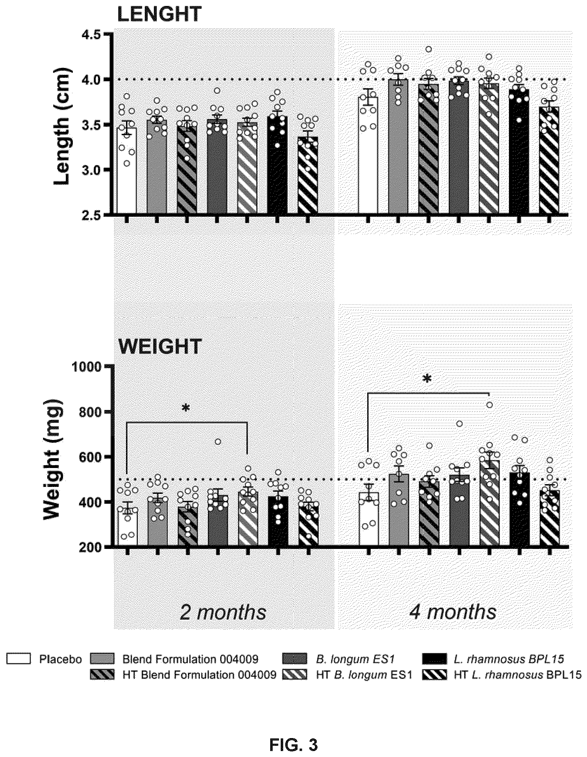

Probiotic and postbiotic ingestion effects on biometrical parameters are shown in Figure 3. Fish length was homogeneous at 2 mpi. At 4 mpi no statistically significant difference was reported although mean values were higher in all postbiotic-fed animals except to HT *L. rhamnosus* BPL15 which was similar to placebo (Table 2).

**Table 2.- Length data (cm) at 2 mpi (2m) and 4 mpi (4m).**

| **2m_P** | **2m_Ble nd** | **2m_HTBIe nd** | **2m_ES1** | **2m_HTE S1** | **2m_BPL 15** | **2m_HTBPL 15** | |
|---|---|---|---|---|---|---|---|
| 3,64 | 3,56 | 3,68 | 3,67 | 3,69 | 3,69 | 3,49 | |
| 3,51 | 3,64 | 3,49 | 3,60 | 3,41 | 3,75 | 3,01 | |
| 3,70 | 3,67 | 3,67 | 3,69 | 3,73 | 3,59 | 3,49 | |
| 3,65 | 3,45 | 3,33 | 3,87 | 3,60 | 3,51 | 3,56 | |
| 3,07 | 3,64 | 3,51 | 3,48 | 3,46 | 3,57 | 3,59 | |
| 3,20 | 3,37 | 3,57 | 3,45 | 3,56 | 3,86 | 3,27 | |
| 3,38 | 3,56 | 3,69 | 3,42 | 3,37 | 3,27 | 3,55 | |
| 3,25 | 3,76 | 3,13 | 3,49 | 3,68 | 3,46 | 3,29 | |
| 3,81 | 3,47 | 3,27 | 3,51 | 3,35 | 3,79 | 3,14 | |
| 3,47 | 3,40 | 3,51 | 3,42 | 3,42 | 3,42 | 3,30 | |
| | | | | | | | |

| | **2m_P** | **2m_Blend** | **2m_HTBIe nd** | **2m_ES1** | **2m_HTE S1** | **2m_BPL15** | **2m_HTBPL 15** |
|---|---|---|---|---|---|---|---|
| Mean | 3,467 | 3,553 | 3,484 | 3,559 | 3,526 | 3,592 | 3,369 |
| Std. Deviati on | 0,2406 | 0,1282 | 0,1878 | 0,1468 | 0,1432 | 0,1837 | 0,1972 |
| Std. Error of Mean | 0,07609 | 0,04053 | 0,05937 | 0,04641 | 0,04529 | 0,0581 | 0,06237 |
| | | | | | | | |

| **4m_P** | **4m_Ble nd** | **4m_HTBIe nd** | **4m_ES1** | **4m_HTE S1** | **4m_BPL 15** | **4m_HTBP L15** | |
|---|---|---|---|---|---|---|---|
| 3,83 | 3,80 | 3,83 | 4,18 | 3,79 | 3,89 | 3,43 | |
| 3,66 | 3,88 | 4,02 | 3,99 | 4,01 | 3,77 | 3,57 | |
| 3,46 | 4,23 | 3,77 | 3,84 | 4,06 | 3,55 | 3,67 | |
| 4,09 | 4,15 | 3,90 | 4,12 | 3,62 | 4,06 | 3,79 | |
| 4,17 | 3,96 | 3,77 | 3,80 | 3,97 | 3,91 | 3,49 | |
| 3,48 | 4,13 | 3,82 | 3,99 | 4,25 | 4,00 | 3,89 | |
| 3,80 | 4,10 | 4,33 | 4,10 | 3,79 | 3,81 | 3,97 | |
| 4,10 | 3,74 | 4,02 | 4,09 | 3,92 | 4,12 | 3,92 | |
| 3,65 | | 4,07 | 3,82 | 4,02 | 4,00 | 3,48 | |
| | | | 3,90 | 4,11 | 3,78 | 3,76 | |
| | | | | | | | |

| | **4m_P** | **4m_Blend** | **4m_HTBIe nd** | **4m_ES1** | **4m_HTE S1** | **4m_BPL15** | **4m_HTBP L15** |
|---|---|---|---|---|---|---|---|
| Mean | 3,804 | 3,999 | 3,949 | 3,984 | 3,955 | 3,888 | 3,698 |
| Std. Deviatio n | 0,2665 | 0,1785 | 0,1826 | 0,1358 | 0,1825 | 0,1671 | 0,1976 |
| Std. Error of Mean | 0,08885 | 0,06312 | 0,06088 | 0,04293 | 0,05772 | 0,05284 | 0,06249 |

Statistical analysis showed a promotion of weight gain at 2 mpi (p=0.0402) and 4 mpi (p=0.0140) in the group HT *B. longum* ES1 compared to Placebo. Similar to length, the rest of the experimental groups showed higher non-significant mean values comparing to the control group at 4 mpi with the exclusion of HT *L. rhamnosus* BPL15 (Table 3).

**Table 3.- Weight data (mg) at 2 mpi (2m) and 4 mpi (4m).**

| **2m_P** | **2m_Ble nd** | **2m_HTBIe nd** | **2m_ES1** | **2m_HTE S1** | **2m_BPL 15** | **2m_HTBP L15** | |
|---|---|---|---|---|---|---|---|
| 451 | 395 | 434 | 446 | 510 | 464 | 391 | |
| 365 | 510 | 386 | 389 | 361 | 469 | 249 | |
| 452 | 478 | 448 | 427 | 476 | 418 | 418 | |
| 441 | 327 | 379 | 667 | 445 | 378 | 451 | |
| 247 | 470 | 256 | 423 | 443 | 480 | 444 | |
| 255 | 412 | 426 | 415 | 411 | 532 | 334 | |
| 358 | 362 | 458 | 392 | 399 | 312 | 340 | |
| 328 | 486 | 281 | 371 | 500 | 335 | 391 | |
| 475 | 412 | 314 | 401 | 366 | 483 | 410 | |
| 369 | 332 | 408 | 373 | 548 | 382 | 377 | |
| | | | | | | | |

| | **2m_P** | **2m_Blend** | **2m_HTBIe nd** | **2m_ES1** | **2m_HTE S1** | **2m_BPL15** | **2m_HTBP L15** |
|---|---|---|---|---|---|---|---|
| Mean | 374,1 | 418,4 | 379 | 430,4 | 445,9 | 425,3 | 380,5 |
| Std. Deviatio n | 81,29 | 65,66 | 71,56 | 86,54 | 62,7 | 71,7 | 60,19 |
| Std. Error of Mean | 25,71 | 20,76 | 22,63 | 27,37 | 19,83 | 22,67 | 19,03 |
| | | | | | | | |

| **4m_P** | **4m_Ble nd** | **4m_HTBIe nd** | **4m_ES1** | **4m_HTE S1** | **4m_BPL 15** | **4m_HTBP L15** | |
|---|---|---|---|---|---|---|---|
| 470 | 524 | 649 | 545 | 629 | 548 | 542 | |
| 563 | 580 | 544 | 564 | 412 | 519 | 388 | |
| 581 | 401 | 445 | 487 | 590 | 582 | 424 | |
| 307 | 607 | 502 | 747 | 514 | 396 | 585 | |
| 293 | 611 | 492 | 416 | 616 | 674 | 476 | |
| 413 | 441 | 524 | 495 | 511 | 686 | 373 | |
| 397 | 638 | 422 | 557 | 652 | 489 | 455 | |
| 563 | 392 | 400 | 413 | 609 | 440 | 406 | |
| 400 | | 436 | 490 | 479 | 541 | 508 | |
| | | | 492 | 830 | 432 | 364 | |
| | | | | | | | |

| | **4m_P** | **4m_Blend** | **4m_HTBIe nd** | **4m_ES1** | **4m_HTE S1** | **4m_BPL15** | **4m_HTBP L15** |
|---|---|---|---|---|---|---|---|
| Mean | 443 | 524,3 | 490,4 | 520,6 | 584,2 | 530,7 | 452,1 |
| Std. Deviatio n | 108,6 | 100 | 76,78 | 94,94 | 115,3 | 97,57 | 74,9 |
| Std. Error of Mean | 36,22 | 35,36 | 25,59 | 30,02 | 36,47 | 30,85 | 23,69 |

### Kinetics

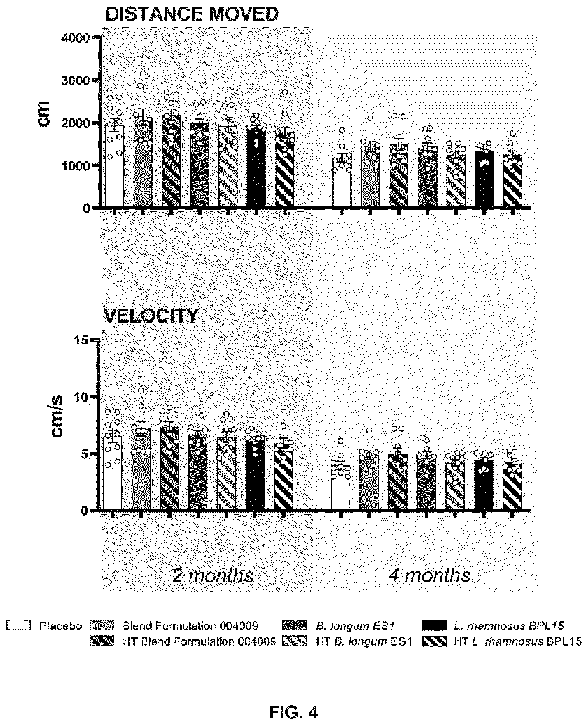

The kinetics of the studied fish showed similar results between groups (Figure 4). Interestingly, the total distance swam by the fish was higher in the 2-month sampling comparing to 4-month one, with distances around 1800-2100 cm (Table 4). The maximum distances correspond to fish included in the groups supplemented with Blend Formulation 004009 and HT Blend Formulation 004009 in both samplings.

**Table 4. Distance moved data (cm) at 2 mpi (2m) and 4 mpi (4m).**

| **2m_P** | **2m_Ble nd** | **2m_HTBIe nd** | **2m_ES1** | **2m_HTE S1** | **2m_BPL 15** | **2m_HTBP L15** | |
|---|---|---|---|---|---|---|---|
| 2336,07 | 2863,3 | 2161,82 | 1727,1 | 1837,66 | 1850,31 | 1595,6 | |
| 1295,29 | 2097,92 | 2718,54 | 2025,75 | 2139,18 | 2032,85 | 1613,21 | |
| 2585,6 | 2195,38 | 1680,44 | 1741,96 | 1386,78 | 1476,73 | 1790,28 | |
| 1610,55 | 1521,24 | 2655,39 | 2428,58 | 2422,06 | 2173 | 1261,83 | |
| 2589,6 | 1527,77 | 2232,37 | 1781,35 | 2080,39 | 1589,78 | 1616,21 | |
| 1673,52 | 2102,33 | 2467,86 | 1870,74 | 2551,99 | 1812,32 | 1749,12 | |
| 2246,22 | 1594,44 | 1811,2 | 2145,8 | 2396,58 | 2084,04 | 1380,16 | |
| 1964,98 | 2761,4 | 2595,32 | 2482,42 | 1459,54 | 1911,36 | 1712,34 | |
| 2020,95 | 3152,98 | 2028,39 | 2121,79 | 1564,09 | 1898,12 | 2219,58 | |
| 1202,3 | 1518,15 | 1511,91 | 1526,72 | 1431,08 | 1918,34 | 2717,91 | |
| | | | | | | | |

| | **2m_P** | **2m_Blend** | **2m_HTBIe nd** | **2m_ES1** | **2m_HTE S1** | **2m_BPL15** | **2m_HTBP L15** |
|---|---|---|---|---|---|---|---|
| Mean | 1953 | 2133 | 2186 | 1985 | 1927 | 1875 | 1766 |
| Std. Deviatio n | 498,3 | 613,1 | 425,4 | 312,8 | 449,8 | 212,3 | 420,8 |
| Std. Error of Mean | 157,6 | 193,9 | 134,5 | 98,91 | 142,2 | 67,14 | 133,1 |
| | | | | | | | |

| **4m_P** | **4m_Ble nd** | **4m_HTBIe nd** | **4m_ES1** | **4m_HTE S1** | **4m_BPL 15** | **4m_HTBP L15** | |
|---|---|---|---|---|---|---|---|
| 1000,89 | 2113,04 | 2164,42 | 1453,18 | 1450,05 | 1525,24 | 1508,39 | |
| 1456,5 | 1559,55 | 2149,9 | 1264,18 | 866,691 | 1436,77 | 1149,8 | |
| 1120,89 | 1452,33 | 1329,69 | 1630,14 | 1521,71 | 1440,72 | 1061,08 | |
| 894,37 | 1178,13 | 1109,79 | 1414,05 | 1111,02 | 1032,66 | 1747,93 | |
| 1239,88 | 1331,79 | 1024,4 | 916,598 | 1383,02 | 1494,09 | 1205,67 | |
| 1097,77 | 1395,21 | 1461,2 | 1377,48 | 1523,16 | 1470,91 | 878,877 | |
| 891,789 | 1478,26 | 1219,8 | 1853,43 | 1378,56 | 1062,15 | 1537,47 | |
| 1829,91 | 1083,64 | 1682,24 | 1384,04 | 727,87 | 1410,41 | 1051,12 | |
| 1140,4 | | 1329,51 | 1870,25 | 1362,54 | 1325,47 | 1344,96 | |
| | | | 1276,46 | 1247,99 | 1084,54 | 1097,05 | |
| | | | | | | | |

| | **4m_P** | **4m_Blend** | **4m_HTBIe nd** | **4m_ES1** | **4m_HTE S1** | **4m_BPL15** | **4m_HTBP L15** |
|---|---|---|---|---|---|---|---|
| Mean | 1186 | 1449 | 1497 | 1444 | 1257 | 1328 | 1258 |
| Std. Deviatio n | 298 | 311,1 | 420,3 | 285,1 | 273,6 | 193,1 | 269,6 |
| Std. Error of Mean | 99,35 | 110 | 140,1 | 90,16 | 86,52 | 61,05 | 85,25 |

Similar to distance data, no statistically significant differences were found between groups when the fish velocity was analysed. In a correlated way, the highest numbers in this parameter corresponded to fish from the groups supplemented with Blend Formulation (Table 5).

**Table 5. Velocity data (cm/s) at 2 mpi (2m) and 4 mpi (4m).**

| **2m_P** | **2m_Ble nd** | **2m_HTBIe nd** | **2m_ES1** | **2m_HTE S1** | **2m_BPL 15** | **2m_HTBP L15** | |
|---|---|---|---|---|---|---|---|
| 7,79313 | 9,71796 | 7,24761 | 5,80187 | 6,20830 | 6,17759 | 5,36515 | |
| 4,31763 | 6,99679 | 9,06906 | 6,76604 | 7,13060 | 6,77617 | 5,37880 | |
| 8,65909 | 7,33751 | 5,70183 | 6,00343 | 4,62938 | 4,92243 | 5,99961 | |
| 5,38214 | 5,20119 | 8,87616 | 8,38249 | 8,08001 | 7,24525 | 4,25604 | |
| 8,63776 | 5,23209 | 7,64616 | 5,97448 | 7,05789 | 5,42737 | 5,38738 | |
| 5,57839 | 7,02275 | 8,34864 | 6,28693 | 8,52140 | 6,07754 | 5,98850 | |
| 7,52653 | 5,31975 | 6,08195 | 7,34258 | 8,00569 | 6,95794 | 4,67090 | |
| 6,60275 | 9,20712 | 8,75143 | 8,28024 | 4,88337 | 6,38227 | 5,71772 | |
| 6,73649 | 10,5394 0 | 6,77756 | 7,07451 | 5,54328 | 6,34568 | 7,43628 | |
| 4,00767 | 5,16378 | 5,08649 | 5,12114 | 4,79263 | 6,43133 | 9,07301 | |
| | | | | | | | |

| | **2m_P** | **2m_Blend** | **2m_HTBIe nd** | **2m_ES1** | **2m_HTE S1** | **2m_BPL15** | **2m_HTBP L15** |
|---|---|---|---|---|---|---|---|
| Mean | 6,524 | 7,174 | 7,359 | 6,703 | 6,485 | 6,274 | 5,927 |
| Std. Deviatio n | 1,67 | 2,032 | 1,419 | 1,073 | 1,475 | 0,6909 | 1,395 |
| Std. Error of Mean | 0,528 | 0,6425 | 0,4487 | 0,3394 | 0,4665 | 0,2185 | 0,441 |
| | | | | | | | |

| **4m_P** | **4m_Ble nd** | **4m_HTBIe nd** | **4m_ES1** | **4m_HTE S1** | **4m_BPL 15** | **4m_HTBP L15** | |
|---|---|---|---|---|---|---|---|
| 3,34613 | 7,05287 | 7,21667 | 4,86272 | 4,8335 | 5,10045 | 5,1785 | |
| 4,86214 | 5,1985 | 7,20475 | 4,22237 | 2,88897 | 4,81942 | 3,85425 | |
| 3,73631 | 5,10377 | 4,45188 | 5,45417 | 5,07237 | 4,80368 | 3,56404 | |
| 2,98203 | 3,94921 | 3,74222 | 4,74576 | 3,75598 | 3,46855 | 5,84749 | |
| 4,18313 | 4,4601 | 3,41968 | 3,05614 | 4,64786 | 5,05238 | 4,19686 | |
| 3,6942 | 4,65192 | 5,04209 | 4,66437 | 5,09078 | 4,90304 | 3,11261 | |
| 2,98257 | 4,92752 | 4,08779 | 6,18386 | 4,60442 | 3,54239 | 5,21461 | |
| 6,13735 | 3,62082 | 5,62545 | 4,62209 | 2,42623 | 4,70135 | 3,53437 | |
| 3,81506 | | 4,44773 | 6,40058 | 4,55211 | 4,50166 | 4,8993 | |
| | | | 4,55227 | 4,17277 | 3,70606 | 3,72285 | |
| | | | | | | | |

| | **4m_P** | **4m_Blend** | **4m_HTBIe nd** | **4m_ES1** | **4m_HTE S1** | **4m_BPL15** | **4m_HTBP L15** |
|---|---|---|---|---|---|---|---|
| Mean | 3,971 | 4,871 | 5,026 | 4,876 | 4,204 | 4,46 | 4,312 |
| Std. Deviation | 1,002 | 1,039 | 1,4 | 0,9626 | 0,9133 | 0,6375 | 0,9093 |
| Std. Error of Mean | 0,334 | 0,3673 | 0,4666 | 0,3044 | 0,2888 | 0,2016 | 0,2875 |

### Zone preference

Taking into account the previously discussed results on the similarity between groups in velocity and distance travelled, the following data are even more interesting.

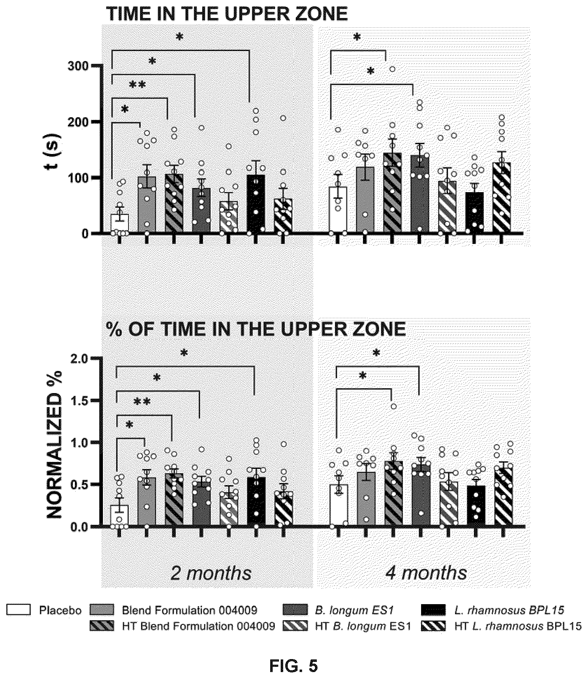

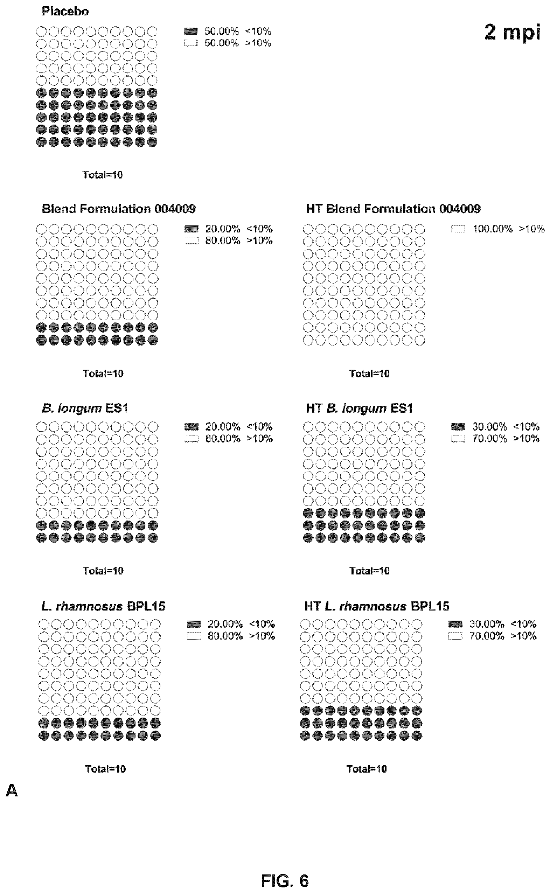

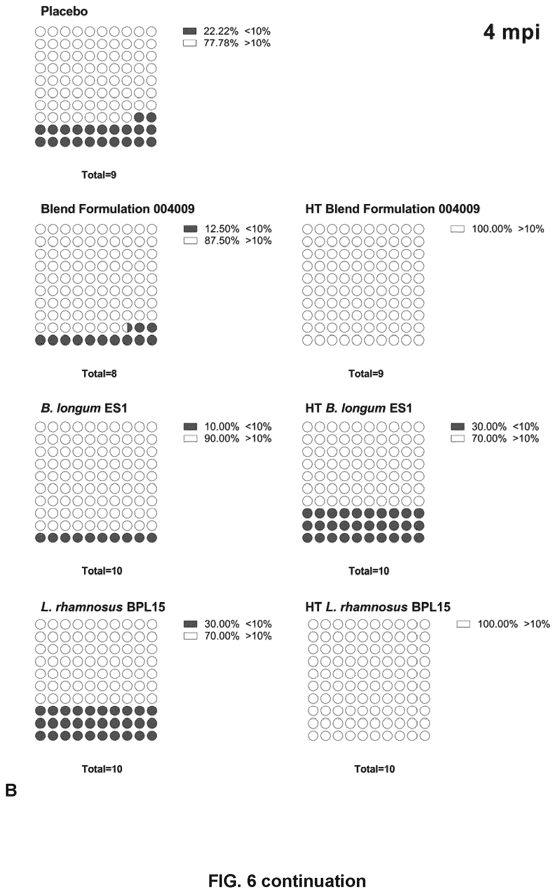

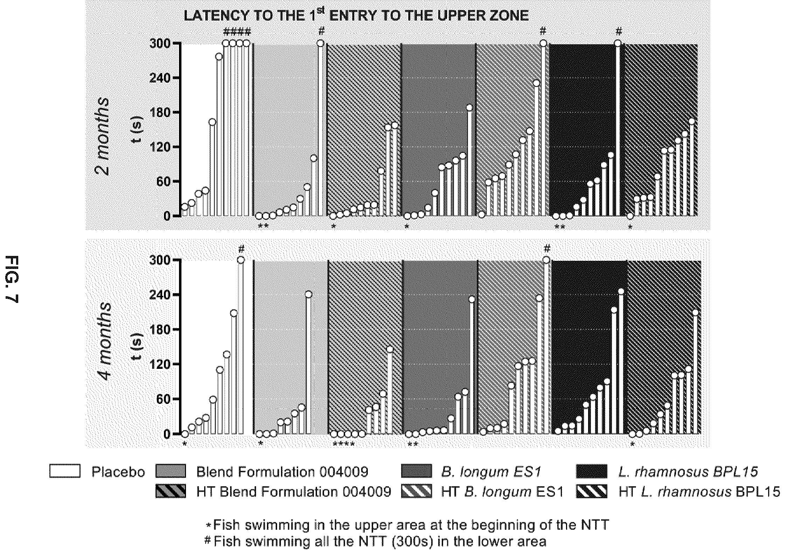

The zone preference in fish has shown a statistically significant preference in several of the experimental groups studied (Figure 5, 6 and 7).

As seen in the graphs (both in the total seconds spent in the upper zone and in the normalized percentage) the data at 2 months sampling are really promising. Four of the groups fed with probiotics or postbiotics reported significant differences compared to the control group:
- Blend Formulation 004009 | p=0.0112
- HT Blend Formulation 004009 | p=0.0024
- *B. longum* ES1 | p=0.0165
- *L. rhamnosus* BPL15 | p=0.0112

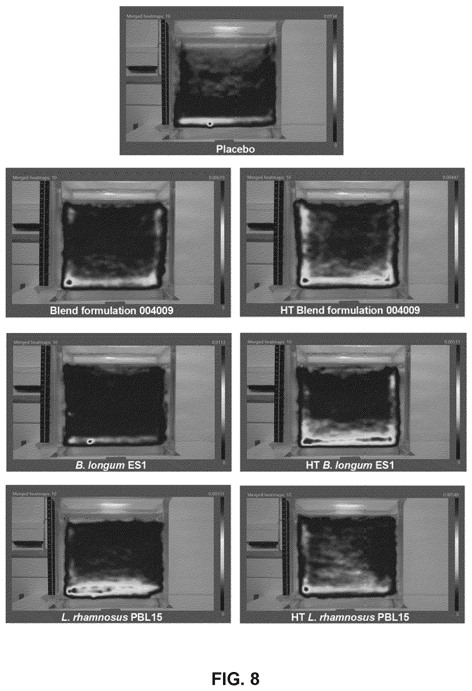

At 2 mpi, the best results (Figure 8, Table 6) were found in the HT Blend Formulation 004009 group with a mean value of 106.2 ±14.98 s in the upper zone, compared to the 34.64 ±12.48 s reported in the placebo group.

**Table 6. Time spent in the upper area (s) at 2 mpi (2m) and 4 mpi (4m).**

| **2m_P** | **2m_Ble nd** | **2m_HTBIe nd** | **2m_ES1** | **2m_HTE S1** | **2m_BPL 15** | **2m_HTBP L15** | |
|---|---|---|---|---|---|---|---|
| 73,36 | 108,6 | 110,56 | 46,6 | 33,24 | 116,8 | 109,92 | |
| 2,28 | 156,04 | 185,76 | 109,24 | 17,64 | 219,36 | 86,4 | |
| 0 | 166,04 | 57,72 | 20 | 84,48 | 72,72 | 0,08 | |
| 37,36 | 62 | 172,92 | 94,12 | 155,24 | 0 | 59,84 | |
| 0 | 164,68 | 83,8 | 83,2 | 42,04 | 93,4 | 206,56 | |
| 89,28 | 79,8 | 71,44 | 71,44 | 48,04 | 7,32 | 40,92 | |
| 93 | 16,16 | 42 | 56,64 | 109,68 | 116,32 | 0,64 | |
| 0 | 0 | 127,84 | 188,84 | 6,12 | 37,84 | 26,76 | |
| 51,08 | 179,04 | 85,84 | 124,92 | 77,76 | 203,68 | 42,4 | |
| 0 | 85,6 | 124,28 | 23,04 | 0 | 180,4 | 44,08 | |
| | | | | | | | |

| | **2m_P** | **2m_Blend** | **2m_HTBIe nd** | **2m_ES1** | **2m_HTE S1** | **2m_BPL15** | **2m_HTBP L15** |
|---|---|---|---|---|---|---|---|
| Mean | 34,64 | 101,8 | 106,2 | 81,8 | 57,42 | 104,8 | 61,76 |
| Std. Deviation | 39,46 | 64,17 | 47,37 | 51,07 | 49,29 | 78,19 | 61,27 |
| Std. Error of Mean | 12,48 | 20,29 | 14,98 | 16,15 | 15,59 | 24,73 | 19,37 |
| | | | | | | | |

| **4m_P** | **4m_Ble nd** | **4m_HTBIe nd** | **4m_ES1** | **4m_HTE S1** | **4m_BPL 15** | **4m_HTBP L15** | |
|---|---|---|---|---|---|---|---|
| 133,88 | 161,52 | 124,12 | 104,32 | 0,6 | 135,44 | 181,6 | |
| 88,88 | 183,4 | 193,96 | 153,6 | 16,76 | 113,12 | 88,56 | |
| 0 | 140,16 | 75,76 | 101,8 | 189,32 | 85,64 | 130,84 | |
| 0,48 | 138,44 | 174,08 | 131,28 | 170,56 | 40,56 | 67,44 | |
| 107,64 | 156,2 | 42,32 | 7,68 | 174,6 | 107,76 | 168,68 | |
| 185,32 | 33,4 | 294 | 192,92 | 95,04 | 4 | 122,16 | |
| 44,64 | 2,2 | 110,16 | 224,6 | 75,28 | 11,52 | 64,68 | |
| 139,24 | 132,88 | 123,2 | 102,88 | 0 | 15,36 | 35,4 | |
| 57,68 | | 156,64 | 234,36 | 106,44 | 119,56 | 196,64 | |
| | | | 139,04 | 111,32 | 107,28 | 207,96 | |
| | | | | | | | |

| | **4m_P** | **4m_Blend** | **4m_HTBIe nd** | **4m_ES1** | **4m_HTE S1** | **4m_BPL15** | **4m_HTBP L15** |
|---|---|---|---|---|---|---|---|
| Mean | 84,2 | 118,5 | 143,8 | 139,2 | 93,99 | 74,02 | 126,4 |
| Std. Deviation | 63,9 | 64,72 | 73,34 | 67,28 | 71,32 | 50,69 | 60,97 |
| Std. Error of Mean | 21,3 | 22,88 | 24,45 | 21,28 | 22,55 | 16,03 | 19,28 |

The results at 4 months post ingestion exclusively provided statistically significant differences in the HT Blend Formulation 004009 group (p=0.0423) and *B*. *longum* ES1 (p=0.0429). These two experimental groups include the fish with the highest percentages in the upper zone of the tank (Figure 8, Table 7) in the sampling.

| **2m_P** | **2m_Blen d** | **2m_HTBI end** | **2m_ES1** | **2m_HTE S1** | **2m_BPL 15** | **2m_HTBP L15** | |
|---|---|---|---|---|---|---|---|
| 0 | 0 | 0,3834696 3 | 0,2611396 4 | 0 | 0 | 0,0163295 7 | |
| 0 | 0,234211 92 | 0,4540453 7 | 0,2807847 2 | 0,143309 15 | 0,156836 61 | 0,0462013 9 | |
| 0 | 0,471895 71 | 0,5097476 2 | 0,4050858 9 | 0,244912 08 | 0,363052 65 | 0,3032712 5 | |
| 0 | 0,541845 42 | 0,5568145 6 | 0,4494635 8 | 0,339317 74 | 0,514740 33 | 0,3782549 5 | |
| 0,087282 97 | 0,563478 48 | 0,5643639 3 | 0,5097476 2 | 0,383662 69 | 0,591895 99 | 0,3853867 5 | |
| 0,360636 29 | 0,645533 24 | 0,6523164 | 0,5545832 1 | 0,411669 58 | 0,672113 43 | 0,3933596 4 | |
| 0,425313 48 | 0,805467 55 | 0,6991825 6 | 0,5944849 4 | 0,534118 49 | 0,673753 75 | 0,4629472 4 | |
| 0,517225 45 | 0,834336 26 | 0,7112031 7 | 0,6477511 | 0,559336 78 | 0,887356 3 | 0,5664274 9 | |
| 0,576974 37 | 0,838893 16 | 0,8620207 1 | 0,7013468 6 | 0,649273 81 | 0,968292 55 | 0,6501042 1 | |
| 0,590455 69 | 0,882731 75 | 0,9056717 1 | 0,9162704 | 0,802799 68 | 1,025638 8 | 0,9786131 2 | |
| | | | | | | | |

| | **2m_P** | **2m_Blend** | **2m_HTBI end** | **2m_ES1** | **2m_HTE S1** | **2m_BPL15** | **2m_HTBP L15** |
|---|---|---|---|---|---|---|---|
| Mean | 0,2558 | 0,5818 | 0,6299 | 0,5321 | 0,4068 | 0,5854 | 0,4181 |
| Std. Deviation | 0,2611 | 0,2883 | 0,1692 | 0,1983 | 0,2407 | 0,3379 | 0,2805 |
| Std. Error of Mean | 0,08255 | 0,09116 | 0,0535 | 0,06272 | 0,07611 | 0,1069 | 0,08871 |

**Table 7. Time spent in the upper area (normalized %) at 2 mpi (2m) and 4 mpi (4m).**

| **4m_P** | **4m_Blen d** | **4m_HTBI end** | **4m_ES1** | **4m_HTE S1** | **4m_BPL 15** | **4m_HTBP L15** | |
|---|---|---|---|---|---|---|---|
| 0 | 0,085734 19 | 0,3850047 9 | 0,1606798 9 | 0 | 0,115720 65 | 0,3506283 5 | |
| 0,040008 05 | 0,340165 93 | 0,5264807 5 | 0,6217817 1 | 0,044733 26 | 0,197222 23 | 0,4828398 5 | |
| 0,395988 2 | 0,728147 15 | 0,6509342 3 | 0,6255785 9 | 0,238603 08 | 0,228235 51 | 0,4939406 | |
| 0,453876 64 | 0,746764 73 | 0,6955270 3 | 0,6306252 9 | 0,524637 38 | 0,376503 41 | 0,5743476 4 | |
| 0,575515 59 | 0,752512 46 | 0,6986414 9 | 0,7227765 8 | 0,597784 53 | 0,563626 84 | 0,6920019 9 | |
| 0,642200 61 | 0,806000 99 | 0,8074693 3 | 0,7487704 1 | 0,638026 63 | 0,640949 2 | 0,7212977 2 | |
| 0,731500 85 | 0,823763 8 | 0,8659349 6 | 0,7973313 | 0,654939 99 | 0,642617 55 | 0,8477516 6 | |
| 0,749438 17 | 0,897589 22 | 0,9340242 5 | 0,9304032 6 | 0,854071 87 | 0,661139 04 | 0,8914444 3 | |
| 0,904162 8 | | 1,4284321 6 | 1,0455444 4 | 0,867691 19 | 0,683167 37 | 0,9433933 7 | |
| | | | 1,0839151 3 | 0,917927 56 | 0,736727 34 | 0,9836610 4 | |
| | | | | | | | |

| | **4m_P** | **4m_Blend** | **4m_HTBI end** | **4m_ES1** | **4m_HTE S1** | **4m_BPL15** | **4m_HTBP L15** |
|---|---|---|---|---|---|---|---|
| Mean | 0,4992 | 0,6476 | 0,7769 | 0,7367 | 0,5338 | 0,4846 | 0,6981 |
| Std. Deviation | 0,3122 | 0,2819 | 0,2964 | 0,2638 | 0,3338 | 0,2324 | 0,2176 |
| Std. Error of Mean | 0,1041 | 0,09966 | 0,09881 | 0,08341 | 0,1055 | 0,07348 | 0,06881 |

The zone preference data was analysed focusing on the number of animals that barely spend time in the upper part of the tank, establishing a threshold of 30 s (10%) of the total NTT (Table 8).

**Table 8. % of time (raw percentage data) during the Novel Tank Test spent in the upper zone. Data have been organized from smallest to largest and a color-coded condition has been applied to easier interpretation. Experimental group code: P) Placebo; Blend) Blend Formulation 0044009; HTBlend) HT Blend Formulation 004009; ES1) B. longum ES1; HTES1) HT B. longum ES1; BPL15) HT L. rhamnosus BPL15; HTBPL15) L. rhamnosus BPL15**

| **2m_P** | **2m_Blend** | **2m_HTBlend** | **2m_ES1** | **2m_HTES1** | **2m_BPL15** | **2m_HTBPL15** |
|---|---|---|---|---|---|---|
| 0,00 | 0,00 | 14,00 | 6,67 | 0,00 | 0,00 | 0,03 |
| 0,00 | 5,39 | 19,24 | 7,68 | 2,04 | 2,44 | 0,21 |
| 0,00 | 20,67 | 23,81 | 15,53 | 5,88 | 12,61 | 8,92 |
| 0,00 | 26,60 | 27,93 | 18,88 | 11,08 | 24,24 | 13,64 |
| 0,76 | 28,53 | 28,61 | 23,81 | 14,01 | 31,13 | 14,13 |
| 12,45 | 36,20 | 36,85 | 27,73 | 16,01 | 38,77 | 14,69 |
| 17,03 | 52,01 | 41,43 | 31,37 | 25,92 | 38,93 | 19,95 |
| 24,45 | 54,89 | 42,61 | 36,41 | 28,16 | 60,13 | 28,80 |
| 29,76 | 55,35 | 57,64 | 41,64 | 36,56 | 67,89 | 36,64 |
| 31,00 | 59,68 | 61,92 | 62,95 | 51,75 | 73,12 | 68,85 |
| | | | | | | |

| **4m_P** | **4m_Blend** | **4m_HTBlend** | **4m_ES1** | **4m_HTES1** | **4m_BPL15** | **4m_HTBPL15** |
|---|---|---|---|---|---|---|
| 0,00 | 0,73 | 14,11 | 2,56 | 0,00 | 1,33 | 11,80 |
| 0,16 | 11,13 | 25,25 | 33,93 | 0,20 | 3,84 | 21,56 |
| 14,88 | 44,29 | 36,72 | 34,29 | 5,59 | 5,12 | 22,48 |
| 19,23 | 46,15 | 41,07 | 34,77 | 25,09 | 13,52 | 29,52 |
| 29,63 | 46,72 | 41,37 | 43,76 | 31,68 | 28,55 | 40,72 |
| 35,88 | 52,07 | 52,21 | 46,35 | 35,48 | 35,76 | 43,61 |
| 44,63 | 53,84 | 58,03 | 51,20 | 37,11 | 35,92 | 56,23 |
| 46,41 | 61,13 | 64,65 | 64,31 | 56,85 | 37,71 | 60,53 |
| 61,77 | | 98,00 | 74,87 | 58,20 | 39,85 | 65,55 |
| | | | 78,12 | 63,11 | 45,15 | 69,32 |

After 2 months of probiotic or postbiotic intake, the lowest percentage of animals showing this type of behaviour is found in the Blend Formulation 004009 and HT Blend Formulation 004009 groups. Contrary, the highest proportion of these individuals was reported in Placebo group.

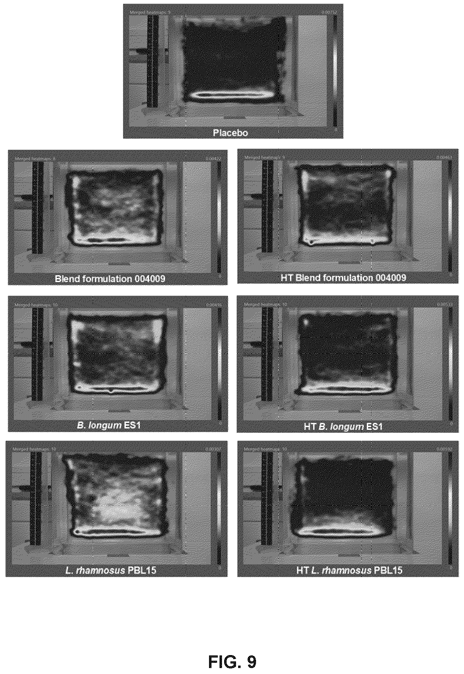

Four months after the start of the experiment, these two groups maintain this trend while the percentage is reduced in *B. longum* ES1 and *L. rhamnosus* BPL15 comparing to the previous sampling (Figure 9).

### Latency to the first entry in the upper zone

Typically, when a fish is introduced to a new tank, its initial preference during the period of acclimatization to the new environment is usually the bottom of the tank.

After 1 minute of acclimatization, the latency shown by the animals in each experimental group was quantified. As can be seen in Figure 9, in the 2-month sampling, in the Placebo group, 4 fish did not score in the upper zone and the latency to rise was delayed above 2 minutes in two of them. In other groups, especially in Blend Formulation 004009, HT Blend Formulation 004009 and B. longum ES1, the latency of the fish at their first entry was globally lower.

After 4 months post probiotic or postbiotic supplementation, the trend softened in the Placebo group although it continued to show lower values in the aforementioned groups, especially in HT Blend Formulation 004009 where up to 4 fish are already in the upper zone at the beginning of the recording after one minute.

### Example 2. EXPERIMENTS IN C. ELEGANS

*C.elegans* has become a good model for study of lifespan, obesity and metabolic syndrome. In addition, it has been used as a simple model to analyze many behaviors, that reveal cognitive impairment (learning and memory, AD, PD), or model aspects of various psychiatric disorders (Dwyer DS. Crossing the Worm-Brain Barrier by Using Caenorhabditis elegans to Explore Fundamentals of Human Psychiatric Illness. Mol Neuropsychiatry. 2018, 3:170-179). In *C. elegans,* neurotransmitters and neuropeptides are similar to the mammalian nervous system (dopamine, GABA, acetylcholine, serotonin, etc.), and the nematode shows a remarkable behavioral plasticity, similar to learning and memory in mammals.

Basic functions such as development, feeding and movement are controlled by the nervous system.

Here, we evaluated a heat-treated postbiotic mix composed by *B. longum* CECT7347 (ES1) and *L. rhamnosus* CECT8361 (BPL15) in the *C. elegans* anxiety and stress models. The mix was evaluated at a final concentration of 1x10⁸ cells/plate (0.5x10⁸ cells/plate HT-BPL15 + 0.5x10⁸ cells/plate HT-ES1).

### Anxiety model

The perception of stressful or repellent stimulus is the first component of anxiety behavior in humans, which in turns is controlled by the amygdala, the HPA (hypothalamic-pituitary-adrenal gland) axis, and neuromodulators (e.g., serotonin).

Serotonin pathway is underlying *C. elegans* aversive behavior, mediating avoidance to the repellent odorant, octanol. Therefore, we used avoidance behavior as an anxiety-related behavior in *C. elegans.*

Anxiety assays were performed by means the avoidance behavior assay. The experiments were carried out using the "smell-on-a-stick" assay previously described (Chao, M. Y., et al. 2004, Proc Natl Acad Sci U S A. 26;101(43):15512-7. doi: 10.1073/pnas.0403369101. Epub 2004 Oct 18. PMID: 15492222; PMCID: PMC524441). "Feeding status and serotonin rapidly and reversibly modulate a *Caenorhabditis elegans* chemosensory circuit." Proceedings of the National Academy of Sciences of the United States of America 101: 15512-15517). For octanol avoidance assays, the blunt end of a hair from a Loew-Cornell (Teaneck, NJ) 9000 Kolinsky 7 paintbrush, taped to a Pasteur pipette, was immersed in freshly prepared 30% octanol and placed in front of a forward-moving nematode, the amount of time it took for the worm to begin backward movement (octanol avoidance) was measured using a laboratory timer.

For the anxiety condition, nematodes were incubated in NGM plates without OP50. This food deprivation provoked an increase of anxiety due to a decrease of levels of serotonin (Chao, M. Y., et al. 2004, Proc Natl Acad Sci U S A. 26;101(43):15512-7. doi: 10.1073/pnas.0403369101. Epub 2004 Oct 18. PMID: 15492222; PMCID: PMC524441). This anxious condition induced an increase of time necessary for the nematode to move backwards the octanol stimulus.

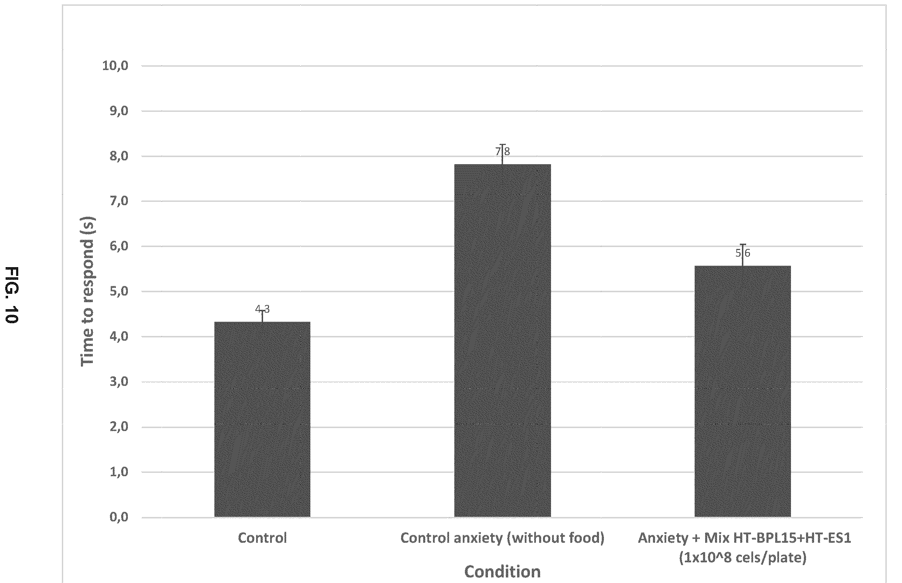

The Figure 10 represents the time spent by nematodes to begin the backwards movement when they smell the octanol. As previously stated, control anxious worms without food, take longer to avoid the octanol than control conditions nematodes with OP50. Nematodes incubated with the mix heat-treated *L. rhamnosus* CECT8361 and heat-treated *B*. *longum* CECT7347 (HT-BPL15+HT-ES1), significantly reduced the avoidance time (p value<0.05) under food deprivation. Therefore, the mix heat-treated *L. rhamnosus* CECT8361 and heat-treated *B. longum* CECT7347 (HT-BPL15+HT-ES1) counteracted the anxiety-related behavior caused by the lack of food.

### Stress model

*C. elegans* locomotion is characterized as sinusoidal waves though muscle contraction, which is mediated by acetylcholine (excitatory neurotransmitter).

In this experiment, we applied a cholinergic stimulation with the drug Denubil, in order to analyse nematode's exploratory behaviour under stress conditions, and evaluate how the heat-treated mix heat-treated *L. rhamnosus* CECT8361 and heat-treated *B. longum* CECT7347 (HT-BPL15+HT-ES1) affected this behaviour pattern.

The plates were divided in three concentric regions, <0.9 cm, 0.9-1.8 cm and 1.8-2.8 cm. The nematodes were placed in the middle of the plate and then scored for its position at the different places after 2 min.

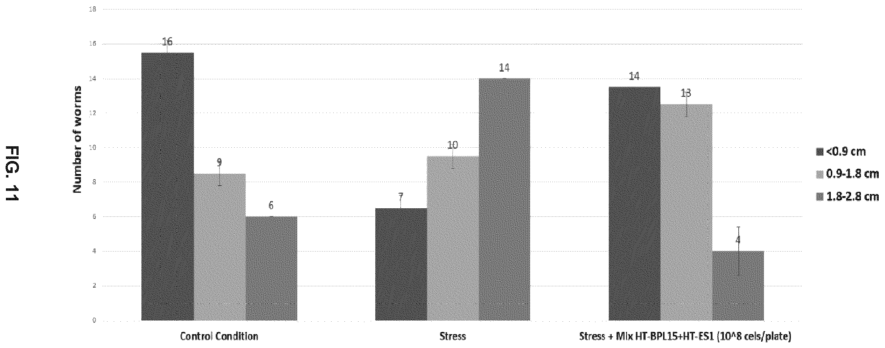

The Figure 11 shows the distribution of worms at fed condition, including both control (not stressed) and stressed populations. Under control conditions, nematodes tend to accumulate in the centre of the plates, while when stressed with the psychostimulant drug, nematodes tend to disperse to the edge of the plates. Moreover, nematodes fed with the mix heat-treated *L. rhamnosus* CECT8361 and heat-treated *B. longum* CECT7347 (HT-BPL15+HT-ES1) restore the behaviour pattern observed in control-non stressed (p value <0.001), again accumulating in the centre of the plates (and not dispersing).

### Example 3. COMPARATIVE ASSAYS AMONG DIFFERENT HEAT-TREATED L. RHAMNOSUS AND B. LONGUM STRAINS BLENDS

### Lactobacillus rhamnosus and Bifidobacterium longum strains

Two strains belonging to *Bifidobacterium longum* (BPL33 and BPL31), and two species from *Lactobacillus rhamnosus* species (BPLA3 and BPL8) were obtained from the applicant's collection, namely Biopolis, S.L. collection, and they were evaluated in the anxiety *C. elegans* model. All the strains were evaluated in its heat-treated form.

Four different combinations were tested:
- *B. longum* HT-BPL33 + *L. rhamnosus* HT-BPLA3
- *B. longum* HT-BPL33+ *L. rhamnosus* HT-BPL8
- *B. longum* HT-BPL31 + *L. rhamnosus* HT-BPLA3
- *B. longum* HT-BPL31+ *L. rhamnosus* HT-BPL8

For comparative analysis, an additional condition with the anxiety blend *B. longum* HT-ES1 + *L. rhamnosus* HT-BPL15 of the invention was included.

### Anxiety model in C. elegans

Octanol avoidance was used as anxiety-related behavior assay in *C*. *elegans.* The experiments were carried out using the "smell-on-a-stick" assay. For octanol avoidance assays, the blunt end of a hair (Loew-Cornell (Teaneck, NJ) 9000 Kolinsky 7 paintbrush) taped to a Pasteur pipette, was immersed in freshly prepared 30% octanol in EtOH (vol/vol) and placed in front of a forward-moving nematode. The amount of time that worms took to move backward was measured. Control condition nematodes were maintained in NGM with *E. coli* OP50. Animals were transferred to NGM plates seeded with 100 µL of freshly overnight *E. coli* OP50. Nematodes were incubated 20 min in these plates before testing. For the anxiety condition, nematodes were incubated during 20 min in NGM plates without OP50. This food deprivation induces anxiety-related behavior due to a decrease of levels of serotonin. To evaluate treatments, wild-type nematodes were age-synchronized in NGM plates supplemented with the corresponding blend of two heat treated strains at 10⁸ cells (final dose). At young-adult stage, nematodes were transferred to NGM plates with each treatment but no food during 30 min. Afterwards, worms were placed in NGM plates without OP50 and with each treatment and tested 20 min later. The percentage of reduction time to respond to octanol in comparison to control anxious is represented.

### Results

The avoidance behavior in response to octanol in *C. elegans* as an anxiety-related behavior was used to evaluate the different blends with alternative *B. longum* and *L*. *rhamnosus* strains.

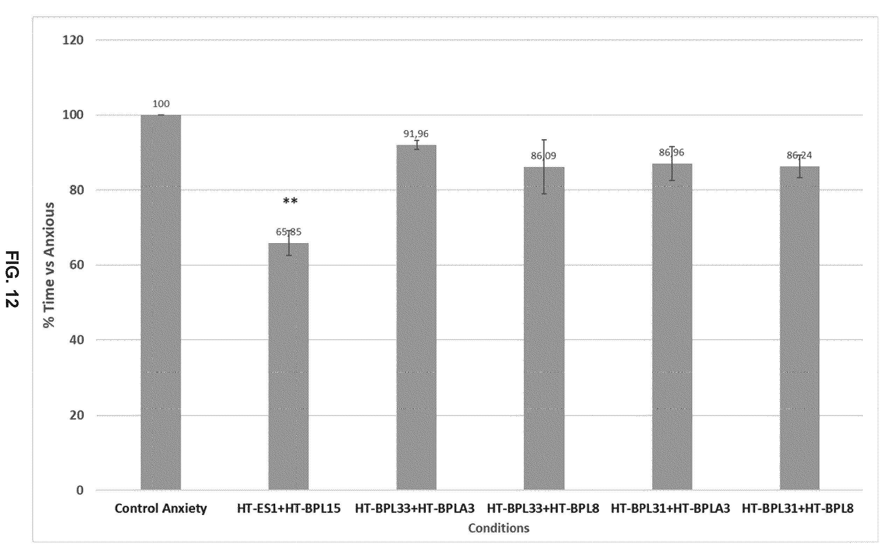

In this assay, anxious control worms take longer to avoid octanol than control conditions nematodes (NGM, control without anxiety). The results represent the relative percentages of each treatment against the anxious control population (Figure 12).

Results indicate that the blend of heat-treated strains with ES1 and BPL15 (the composition of the invention) provided 34% of reduction of the time of response when the octanol was presented to the worms, showing a high positive effect reducing the anxious behavior while the other four blends only reduced between 8 and 14% (Figure 12 and Table 9).

**Table 9.** Percentage of reduction time to avoidance behavior (anxiety model) in *C*. *elegans* obtained with the different blends included in this study. HT-ES1 + HT-BPL15 blends on avoidance behavior.

| **Strain Combination** | **% Reduction time to respond to octanol (sec) vs. control anxious** |
|---|---|
| HT-ES1 + HT-BPL15 (1x10⁸ cells) | 34.15 |
| HT-BPL33 + HT-BPLA3 (1x10⁸ cells) | 8.40 |
| HT-BPL33 + HT-BPLA8 (1x10⁸ cells) | 13.91 |
| HT-BPL31 + HT-BPLA3 (1x10⁸ cells) | 13.40 |
| HT-BPL31 + HT-BPL8 (1x10⁸ cells) | 13.76 |

Therefore, these results indicate that the blend of strains of the present invention has a surprisingly better effect than other blends of strains of these species.

## Claims

1. Postbiotic composition comprising non-viable *Bifidobacterium longum* strain CECT 7347 and non-viable *Lactobacillus rhamnosus* strain CECT 8361.

2. Postbiotic composition according to claim 1, wherein the non-viable *Bifidobacterium longum* and *Lactobacillus rhamnosus* strains are heat-treated.

3. Postbiotic composition according to claim 1 or 2, wherein the postbiotic composition is a pharmaceutical composition or a nutritional composition.

4. Postbiotic composition according to claim 3, wherein the pharmaceutical composition comprises a pharmaceutically acceptable carrier and/or an excipient.

5. Postbiotic composition according to any of claims 3 or 4, wherein the composition is formulated in liquid form or in solid form.

6. Postbiotic composition according to claim 5, wherein the solid formulation is selected from the group consisting of tablets, lozenges, sweets, chewable tablets, chewing gum, capsules, sachets, powders, gels, granules, coated particles or coated tablets, tablets and gastro-resistant tablets and capsules and dispersible strips and films.

7. Postbiotic composition according to claim 5, wherein the liquid formulation is selected from the group consisting of oral solutions, suspensions, droplets, emulsions and syrups.

8. Postbiotic composition according to claim 3, wherein the nutritional composition is a food or a nutritional supplement.

9. Postbiotic composition according to claim 8, wherein the food is selected from the group consisting of fruit or vegetable juices, ice cream, infant formula, milk, yogurt, cheese, fermented milk, powdered milk, cereals, baked goods, milk-based products, meat products and beverages.

10. Postbiotic composition according to any one of claims 1 to 9, wherein the total concentration of *B. longum* and *L. rhamnosus* in the composition is between 10³ and 10¹² cells.

11. Postbiotic composition according to any one of claims 1 to 10, for use as a medicament.

12. Postbiotic composition according to any one of claims 1 to 10, for use in the treatment and/or prevention of anxiety disorders.

## Patentansprüche

1. Postbiotische Zusammensetzung, die den nicht lebensfähigen *Bifidobacterium longum-Stamm* CECT 7347 und den nicht lebensfähigen *Lactobacillus rhamnosus-Stamm* CECT 8361 umfasst.

2. Postbiotische Zusammensetzung nach Anspruch 1, wobei die nicht lebensfähigen *Bifidobacterium longum-* und *Lactobacillus rhamnosus-Stämme* hitzebehandelt sind.

3. Postbiotische Zusammensetzung nach Anspruch 1 oder 2, wobei die postbiotische Zusammensetzung eine pharmazeutische Zusammensetzung oder eine Nährstoffzusammensetzung ist.

4. Postbiotische Zusammensetzung nach Anspruch 3, wobei die pharmazeutische Zusammensetzung einen pharmazeutisch unbedenklichen Träger und/oder einen Hilfsstoff umfasst.

5. Postbiotische Zusammensetzung nach einem der Ansprüche 3 oder 4, wobei die Zusammensetzung in flüssiger Form oder in fester Form formuliert ist.

6. Postbiotische Zusammensetzung nach Anspruch 5, wobei die feste Formulierung ausgewählt ist aus der Gruppe bestehend aus Tabletten, Lutschtabletten, Bonbons, Kautabletten, Kaugummi, Kapseln, Beuteln, Pulvern, Gelen, Granulaten, überzogenen Partikeln oder überzogenen Tabletten, Tabletten und magenresistenten Tabletten und Kapseln sowie dispergierbaren Streifen und Folien.

7. Postbiotische Zusammensetzung nach Anspruch 5, wobei die flüssige Formulierung ausgewählt ist aus der Gruppe bestehend aus oralen Lösungen, Suspensionen, Tropfen, Emulsionen und Sirupen.

8. Postbiotische Zusammensetzung nach Anspruch 3, wobei die Nährstoffzusammensetzung ein Lebensmittel oder ein Nahrungsergänzungsmittel ist.

9. Postbiotische Zusammensetzung nach Anspruch 8, wobei das Lebensmittel ausgewählt ist aus der Gruppe bestehend aus Frucht- oder Gemüsesäften, Eiscreme, Säuglingsnahrung, Milch, Joghurt, Käse, fermentierter Milch, Milchpulver, Cerealien, Backwaren, Produkten auf Milchbasis, Fleischprodukten und Getränken.

10. Postbiotische Zusammensetzung nach einem der Ansprüche 1 bis 9, wobei die Gesamtkonzentration von *B*. *longum* und *L. rhamnosus* in der Zusammensetzung zwischen 10³ und 10¹² Zellen liegt.

11. Probiotische Zusammensetzung nach einem der Ansprüche 1 bis 10 zur Verwendung als Medikament.

12. Probiotische Zusammensetzung nach einem der Ansprüche 1 bis 10 zur Verwendung bei der Behandlung und/oder Vorbeugung von Angststörungen.

## Revendications

1. Composition postbiotique comprenant la souche *Bifidobacterium longum* non viable CECT 7347 et la souche *Lactobacillus rhamnosus* non viable CECT 8361.

2. Composition postbiotique selon la revendication 1, dans laquelle les souches *Bifidobacterium longum* et de *Lactobacillus rhamnosus* non viables sont traitées thermiquement.

3. Composition postbiotique selon la revendication 1 ou 2, dans laquelle la composition postbiotique est une composition pharmaceutique ou une composition nutritionnelle.

4. Composition postbiotique selon la revendication 3, dans laquelle la composition pharmaceutique comprend un véhicule et/ou un excipient pharmaceutiquement acceptable.

5. Composition postbiotique selon l'une quelconque des revendications 3 ou 4, dans laquelle la composition est formulée sous forme liquide ou solide.

6. Composition postbiotique selon la revendication 5, dans laquelle la formulation solide est choisie dans le groupe constitué par les comprimés, pastilles, bonbons, comprimés à mâcher, chewing-gums, gélules, sachets, poudres, gels, granulés, particules enrobées ou comprimés enrobés, comprimés et comprimés et gélules gastro-résistants et bandes et films dispersibles.

7. Composition postbiotique selon la revendication 5, dans laquelle la formulation liquide est choisie dans le groupe constitué par les solutions orales, suspensions, gouttelettes, émulsions et sirops.

8. Composition postbiotique selon la revendication 3, dans laquelle la composition nutritionnelle est un aliment ou un complément nutritionnel.

9. Composition postbiotique selon la revendication 8, dans laquelle l'aliment est choisi dans le groupe constitué par les jus de fruits ou de légumes, crème glacée, préparations pour nourrissons, laits, yaourt, fromage, lait fermenté, lait en poudre, céréales, produits de boulangerie, produits à base de lait, produits à base de viande et boissons.

10. Composition postbiotique selon l'une quelconque des revendications 1 à 9, dans laquelle la concentration totale en *B*. *longum* et *L*. *rhamnosus* dans la composition est comprise entre 10³ et 10¹² cellules.

11. Composition postbiotique selon l'une quelconque des revendications 1 à 10, destinée à être utilisée en tant que médicament.

12. Composition postbiotique selon l'une quelconque des revendications 1 à 10, destinée à être utilisée pour le traitement et/ou la prévention de troubles anxieux.
